# EUROPEAN PATENT SPECIFICATION

(11) **EP 3 238 111 B1**
(45) Date of publication and mention of the grant of the patent: **05.02.2025**
(21) Application number: 15839067.4
(22) Date of filing: 23.12.2015
(51) Int. Cl.: G16H 10/40

(54) **SYSTEM AND METHOD FOR ADAPTIVE MEDICAL DECISION SUPPORT**
SYSTEM UND VERFAHREN FÜR ADAPTIVE MEDIZINISCHE ENTSCHEIDUNGSUNTERSTÜTZUNG
SYSTÈME ET PROCÉDÉ POUR LA PRISE DE DÉCISION ADAPTIVE MEDICALE

(30) Priority: 24.12.2014 US 201462096746 P
(43) Date of publication of application: 01.11.2017
(73) Proprietor: Genomate Health Inc., Cambridge, MA 02142 (US)
(72) Inventor: PETAK, Istvan, 125 Budapest (HU); SCHWAB, Richard, Erno, 1022 Budapest (HU)
(74) Representative: Bartle Read
(86) International application number: PCT/IB2015/002547
(87) International publication number: WO 2016/103036

(56) References cited:
- US-A1- 2011 202 361
- US-A1- 2012 016 206
- US-A1- 2013 268 290
- US-A1- 2014 081 659
- ANONYMOUS ET AL: "Masking (Electronic Health Record)", WIKIMEDIA, 11 November 2014 (2014-11-11), XP055270299, Retrieved from the Internet <URL:https://en.wikipedia.org/w/index.php?title=Masking_(Electronic_Health_Record)&oldid=633318567> [retrieved on 20160503]
- ANONYMOUS: "Medical privacy", WIKIPEDIA, 23 June 2014 (2014-06-23), XP055270301, Retrieved from the Internet <URL:https://en.wikipedia.org/w/index.php?title=Medical_privacy&oldid=614108346> [retrieved on 20160503]

## Description

### FIELD OF THE INVENTION

Embodiments of the present invention relate to an adaptive treatment development tool, specifically a system and method for an adaptive medical decision support.

### BACKGROUND

In the new era personalized medicine, there is a need to adapt treatment to each patient based on each patient's "story." There is an advantage to learning and sharing these stories in development of treatments across patient populations. A system for automated sharing and adapting treatments accordingly for each individual patient would be advantageous, but does not exist.

The human genome project has explored millions of genetic alterations. The Catalogue of Somatic Mutations of Cancer contains 2.1 million different variants in 550 cancer genes. Each tumor can contain a combination of up to 8 cancer genes. This means that more than 50% of cancer patients have cancer mutations less frequent than 1%.

There are hundreds of targeted therapies in development. Newest technologies enable personal genomics to become a commodity. However, there is no information technology solution to link genetic alterations to prognosis and therapy. The large number of genetic variations is a great challenge, but the analytical reproducibility of genetic data is a unique opportunity to share experience.

### BRIEF SUMMARY OF THE INVENTION

The present invention is defined by the appended claims. Accordingly, the present application is directed to a method to treat human diseases based on therapy ranking in a linked, learning database of the clinical experience generated by others who have used an adaptive decision support system that obviates one or more of the problems due to limitations and disadvantages of the related art.

This invention relates generally to techniques for artificial intelligence based medical decision support systems. More particularly, it relates to assigning ranks to treatment options based on their expected efficacy and side effects and clinical experience. More particularly, it relates to the clinical annotation of rare genetic variants, treatments, and response to treatments. More particularly, it relates to the treatment of patients based on this annotation, and sharing the result with others who use the decision support system.

An advantage of the present invention is to provide a system and method for adaptive medical decision support.

The present invention contemplates virtually instant sharing of clinical experience with all users who use the same decision support system. This reduces the time to learn from each patient's "story" for the whole medical community. This avoids the repetition of a non-effective treatment for the next patient with the same medical condition if the system is used to choose treatment, and helps to choose the effective treatment if such experience is already available. Further, this decision support system can be used directly by the end-users (physicians, patients) without any time delay by involving additional person who operates the decision support system or a team of persons who have to analyze data retrospectively to change the recommendation of a decision support system.

Additional features and advantages of the invention will be set forth in the description which follows, and in part will be apparent from the description, or may be learned by practice of the invention. The objectives and other advantages of the invention will be realized and attained by the structure particularly pointed out in the written description and claims hereof as well as the appended drawings.

To achieve these and other advantages and in accordance with the purpose of the present invention, as embodied and broadly described, in one embodiment, an adaptive medical treatment decision system includes a medical experience register, the medical experience register storing medical experience data from a plurality of users, wherein the data from each user is encoded and includes anonymous, patient-specific physical, biological and clinical data and; a clinical evidence register, the clinical evidence register storing result data from at least a subset of the plurality of users, the result data including anonymous molecular and clinical profiles of respective prior patients, diagnoses of the respective prior patients, treatments administered to the respective prior patients and outcomes of the treatments administered to the respective prior patients; a processor connected to the medical experience register and the clinical evidence register for requesting and receiving signals from the medical experience register and the clinical evidence register, the processor enabled to process said received signals to rank treatment outcomes based on a specific patient physical, biological or clinical profile and diagnosis.

In another aspect of the present invention, another embodiment of the system and method for adaptive medical decision support includes a method of assigning preference rank to treatment options for a specific patient, comprising continually ranking a plurality of treatments for a plurality of corresponding diseases, said ranking based on a plurality of information specific to each of a plurality of patients, wherein said information includes diagnosis, genetic profile, treatment and outcome for each patient of the plurality of patients, said continual ranking performed by a special processor for receiving the plurality of information and corresponding metadata; receiving at the special processor specific descriptors of the specific patient's condition, the specific descriptors including a diagnosis and a specific genetic profile of the specific patient; and the special processor generating at least one treatment profile based on the continual ranking of the plurality of treatments, the at least one treatment profile having a high ranking for the specific patient based on the specific descriptors at the time the at least one treatment profile is generated. Although, there are many ongoing great public and private efforts to link existing clinical and genetic databases, none of them enables the users to interpret the complex clinical and molecular diagnostic findings themselves, make their own decisions based on the evidence they consider important and on the clinical experience of others, and contribute to the community by sharing their clinical experience instantly in real time, within seconds.

One aspect of the present invention uses the active participation of patients and people who want to fight cancer and their contribution has to be integrated into the one common effort against cancer.

Further embodiments, features, and advantages of the system and method for adaptive medical decision support, as well as the structure and operation of the various embodiments of the system and method for adaptive medical decision support, are described in detail below with reference to the accompanying drawings.

Publication US2013/268290A1 is the closest prior art and discloses a ranking of treatment options based on genetic variants. But said document does not disclose a registration of the clinical responses of the ranked treatment options, and also does not disclose a cascaded use of data from patients with the same driver genetic alterations, and alternatively from patients with alterations in the same driver genes, and alternatively from patients with driver genes in the same transduction pathway, and alternatively from patients with the same tumor type.

### BRIEF DESCRIPTION OF THE DRAWINGS

The accompanying figures, which are incorporated herein and form part of the specification, illustrate a system and method for an adaptive medical decision support according to aspects of the present invention. Together with the description, the figures further serve to explain the principles of the system and method described herein and thereby enable a person skilled in the pertinent art to make and use the system and method for adaptive medical decision support.
FIG. 1 is an exemplary schematic illustration of a data sharing architecture according to one embodiment of the present invention.
FIG. 2 is a rudimentary representation of feedback within the data sharing structure according to one embodiment of the present invention.
FIG. 3 represents a rudimentary exemplary treatment calculation according to aspects of the present invention.
FIG. 4 is illustrates a rudimentary relationships and weighting in a treatment calculation according to aspects of the present invention.
FIG. 5 is a schematic illustration of a hardware infrastructure according to the present invention.
FIG. 6 is basic illustration of the steps of the method of utilizing the molecular treatment calculator according to aspects the present invention.
FIGS. 7-10 provide an examples of patient data and treatment profiles according to the present system.
FIGS. 11-15 are exemplary illustrations of various user interfaces available according to aspects of the present invention.

### DETAILED DESCRIPTION OF THE INVENTION

Reference will now be made in detail to embodiments of the system and method for adaptive medical decision support with reference to the accompanying figures, in which like reference numerals indicate like elements.

Embodiments of the present invention relate to an internet-based computer program that links genetic alterations to clinical experience and may be used by any physician, patient or anybody who is searching for and willing to share medical information. Users of this program may share experience, make online consultations, and use calculators to assess risk, prognosis and treatment options. An adaptive database of methods is generated. The database may be modified by the addition of diagnoses, methods, treatments, experience and other information pertinent to treatment decisions and outcomes. The database may be shared by users, who provide the input about the descriptors, e.g., frequency of genetic variations, response to therapies in association with specific genetic alterations by using the system and method to calculate treatment options or cancer risk.

Initially, before the database can be populated with sufficient direct real-life experience with responses to treatments in case of a majority of genetic variations, the adaptive database may rank the most likely effective therapy based on published evidence as the highest rank recommendation. Thereafter, the adaptive database model can be built and populated with evidence generated by the system (e.g., input by users, diagnostic devices or other methods), thus supporting the "best" decision based on the molecular profile, for example, evidence for most likely driver genes and mutations, associations between driver cancer genes and targets and targets and drugs. A "best" decision according the adaptive database can be defined as the treatment with the most clinical experience, highest evidence or least side effects, which influences the physician's ultimate treatment decision, based on the patient current situation and preferences. Other factors may influence what is the "best" decision as information populates the system, as can be appreciated by one of skill in the art.

The present system provides functionality not previously available because the present system introduces diagnostic treatment and outcome tracking tools and other components, and integration thereof, to provide a new, not previously known functionality. The present system transforms health data and diagnostic signals into actual adaptive knowledge in a data sharing architecture that will be detailed herein.

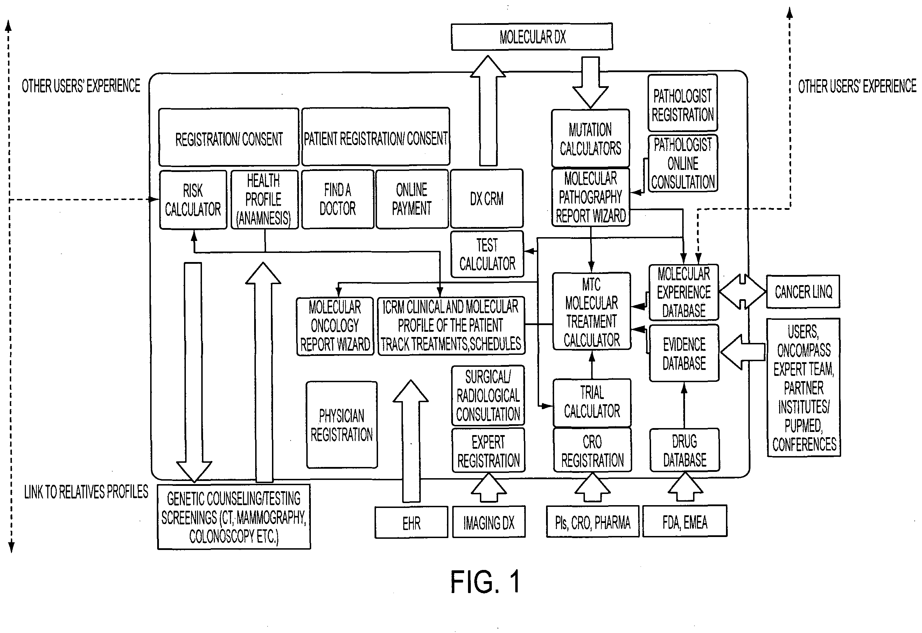

An exemplary architecture of a system according to the present invention is shown at FIG. 1. As can be seen in FIG. 1. the Molecular Treatment Calculator (MTC) receives inputs from an Experience Database, Evidence Database, Molecular Pathology Report "wizard", and Trial Calculator, each of which is described in further detail below. The Experience Database may be a Clinical Experience Database or may include other experience information as may be relevant to the present system. Each of these "input components" receives its own set of inputs from various sources, as illustrated in FIG. 1. and described in more detail below.

As is illustrated, the MTC is the ultimate receiver of many inputs upstream in the architecture. In some instances, the inputs are generated automatically by medical device hardware, for example, hardware devices used in molecular diagnostics, genetic testing, laboratory automats, CT imaging, mammography, x-ray imaging, colonoscopy results, etc. Each of the inputs from these devices may be standardized to include objective information in an encoded signal, with identifying header/metadata to be received and processed by the system, and translated to weighted inputs to the MTC.

In one aspect of the present invention, the molecular treatment calculator (MTC), which combines scientific evidence-based and clinical experience-based decision support, may only work with direct input only from molecular diagnostics. In such case, the molecular treatment calculator may be connected to other types of diagnostics, e.g., imaging, and receive feed-back from treatment outcomes from a patient CRM (customer relationship management) or EHR (electronic health record system) built in the system or from other EHR system, which can provide such data. In such case, there may be a whole platform in which the treatment calculator is embedded in the clinical practice with connections to other medical applications. In some instances, the MTC may receive input from as few as one source calculator, e.g. the scientific evidence calculator or the clinical experience calculator, or may receive input from multiple calculators, such as the scientific evidence calculator and the clinical experience calculator. Other information from other sources, as exemplified by FIG. 1, may also be used by the MTC in decision support.

The present system thus provides an internet-based personal precision medicine expert system ("Precision Medicine Calculator or Realtime Oncology Treatment Calculator"), which links genomics to clinical experience of physicians and patients to use the power of the cooperation of the global human community against cancer. Aspects of the present invention allow a physicians to be "an expert" that interprets the molecular cancer profile independently and makes an individual treatment decision, to find "a cure" based on evidence selected by the physician, incorporating his and his colleagues' clinical experience, to share experience with his colleagues both locally and across the user base, and to act locally based on broad-based, multi-platform inputs. Patients accessing the system have the opportunity to find the best specialist to help, access to a cure for similar cases that might otherwise only be known in another area of the world and share their results to improve the treatment of others (and contribute to the world for cancer research). Non-cancer patients, such as those with risk factors, can take control now, including identifying risk factors otherwise not known, be part of a community against cancer and join the fight by contributing data and calculate their own or their loved ones' risk to prevent cancer and find best practices to prevent cancer.

The system consists of registration modules for different users (healthy users, patients, physicians, pathologist, experts (surgeons etc.)) all users use the same system, but have different preferences to access or enter data in certain applications. Healthy users use risk calculators to enter data about their biomedical parameters. Based on the risk, the user receives a personalized prevention plan and healthcare providers participating in prevention (e.g. cancer prevention) are offered. These healthcare providers are users of the system and directly link diagnostic results to the database of the healthy user health record (Health Profile (anamnesis)). This experience is shared with the risk calculator to help others in risk assessment. For example, genetic and environmental factors are linked to cancer risk since these factors in the users' medical history are automatically linked to cancer (and type and molecular profile of cancer) and the age of the users when (if) cancer is diagnosed in the users.

The precisely documented clinical history helps the risk assessment of relatives, especially in case of family anamnesis. If a user is diagnosed with a disease - e.g. cancer - the user becomes a patient user. Any new patient user can also register. The system will offer the physician users showing their professional profile as partner physicians and healthcare providers. Users can also pay for services through the system. If the patient user was previously a healthy user, all anamnesis data is used in the built in CRM system. The patient personal data is encrypted. The biomedical profile (clinical and molecular profile) is stored in the central CRM system.

This anamnesis and previous molecular profile provided by the CRM, knowledge about the frequency of genetic alterations, and knowledge of the MTC (molecular treatment calculator), which genetic alterations can alter therapy ranking is used by the Test Calculator.

The molecular diagnostic test can be ordered by sharing the patient information with the diagnostics provider user. The diagnostics provider can use the built in diagnostics process CRM and then the molecular alterations calculators enter data. The pathologist user can use the system to generate pathology report-by-report wizard.

The molecular profile provides information to the molecular treatment calculator for therapy ranking for the particular patient and provides information to the clinical experience database, which in turn provides information for the evidence database. These databases are used by the molecular treatment calculator. The evidence database receives information about drugs from public databases.

The clinical experience database/calculator also receives input from other users' system as well and third party systems like Cancer LINQ (Learning Information Network for Quality). The evidence database is built also with published evidence by users and curated by the editors of the database.

The clinical experience database also receives clinical outcome data from the CRM system.

The trial calculator is built by the physician users who are principal investigators of clinical trials, clinical research organizations (CRO), and pharmaceutical companies' clinical development units. The trial calculator provides information to the molecular treatment calculator about the available compounds in clinical trials considering the clinical characteristics and medical history of the particular patient received from the CRM system. Users may access and search the trial calculator to identify available trials based on search criteria. Physicians may use the trial calculator to refer a patient to the identified trial, or a patient may use the trial calculator as a mechanism for requesting participation in the trial. Inversely, the trial calculator may also allow clinical trial operators to identify patients as possible participants in a trial.

The users can ask for online consultation with surgeons, radiologist etc. experts about the evaluation of clinical response and possibility of surgical procedures.

The system's CRM can receive information from third party EHR systems, but the system can work completely independently as well.

The physician user who makes the recommendation about the treatment strategy generates a calculator report (a Precision Medicine Report) based on the molecular treatment calculator and the online consultations. The treatment decisions and clinical responses are registered in the CRM system.

A method, which is used by a group of people linked together in a network for treatment of human diseases, to assign preference rank to treatment option based on the similarity of the given patient's case to cases treated by the users of the same method previously. The ranking is based on the efficacy of treatments on patients, which are most similar to the case. Similarity is based on the number of matching parameters. This way the first therapy is the most effective in patients, which are most similar to this case.

The overall similarity is calculated by the combined similarity based on the specific descriptors of the patient. Examples for these descriptors without limitation are the diagnosis of the disease, the laboratory parameters and genetic background of the patient. A database or register of this method is generated and shared by users, who provide the input about the descriptors, e.g. frequency of genetic variations, response to therapies in association with specific genetic alterations by using the system to calculate treatment options or cancer risk.

In an aspect of the present invention, targeted cancer therapy, the main descriptors are the molecular genetic variations found in the patient's cancer.

In another aspect, the user is a medical professional. In another aspect the user is a patient. Drugs are ranked based on their efficacy and side effects in patients with the given combination of genetic variations in the clinical experience of the users of the system. The patient is treated based on this ranking and the clinical response is registered in the system to add to the experience database or register. The personal data of the patients may be restricted so as to be only available to the treating physicians, but the clinical and molecular parameters linked to the response to therapy may be shared in a common database.

In case of parameters, e.g., genetic variations, which can alter the drug sensitivity, but are unknown in the particular patient, the treatment rank may be based on the frequency of the unknown parameter based on the experience with patients with similar characteristics in the shared database or register.

The tests of genetic alterations may be ranked based on their ability alter the ranking of the therapy and their frequency of alterations in the database/register generated by the users. The required tests can be performed and the results are registered in the database/register of the system.

The system combines published evidence and evidence generated during the molecular interpretation and ranking of treatment options in case of genetic alterations when direct experience is limited. The system enables physician users to add published evidence, share this with others, disregard publish evidence. Published evidence is ranked by the impact factor of the journal and the frequency "citation" of the users of the system. The system enables patient and physician users to combine experiences and work together to build a shared knowledgebase.

### Database Architecture

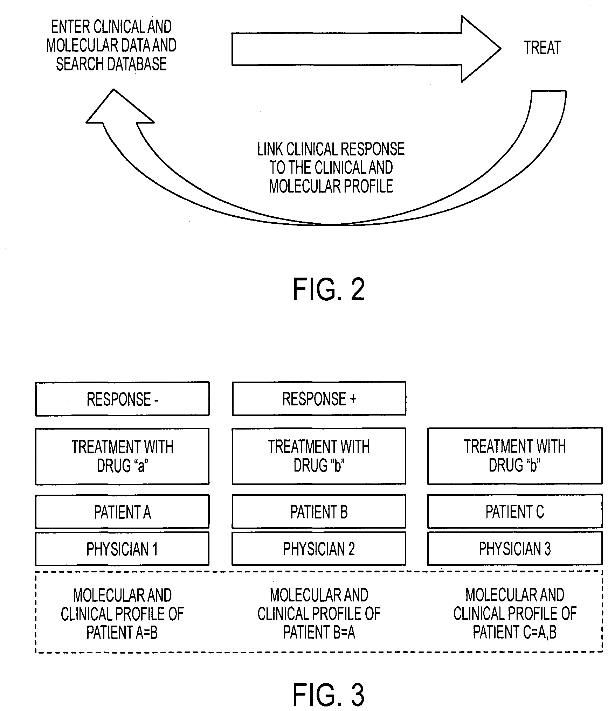

As illustrated in FIG. 2, it is contemplated that users, physicians and patients will register and consent if they are willing to share their or their patients' physical, biological, clinical data with the central database/register for the benefit of others who use the same system, and third parties who may want to do biomedical research in the database/register. In such case, the personal data of patients is encrypted and accessible only for their physician, and all medical parameters are anonymous for the central database/register. But all biomedical data related to the same person will remain linked together in the biomedical identity of the patient.

The basic concept is the "use it share it." Users are not asked to enter any data to simply to contribute to the database/register. User may choose to receive inputs back for their own use and benefit. Users use applications like "calculators" and online consultation modules. These applications use standardized data as input information, which helps to build a searchable database/register. The users have to consent that they are willing to share their physical, biological, clinical data (without personal data) for the benefit of others in the database/register of the their physician or in the shared experience database/register of all users. More specifically the concept applies to physicians who treat a cancer patient with a rare genetic alteration. The physician uses the system to choose a therapy and then the clinical results of the treatment (both positive and negative) is registered in the system's central database/register.

In this way, if another patient is diagnosed somewhere in the world with the same rare mutation, his or her doctor can search the database/register 1 second later and immediately learn from the experience of this previous case similar to the present case. But, while searching the entered genetic and clinical data is stored therefore it contributes to the treatment of the next patients. As illustrated in FIG. 3, a later user physician can learn from prior outcomes/responses of patients having the same molecular and clinical profile, but different treatments. Referring to FIG. 3, it can be seen that Patient A and Patient B have the same molecular and clinical profile, but are treated by different physicians (Physician 1 and Physician 2) with different drugs (drug "a" and drug "b"). The response of Patient A to drug "a" is characterized as negative, and the response of Patient B to drug "b" is characterized as positive in the database. As a result, Physician 3 treating Patient C, who has the same molecular and clinical profile as Patients A and B, will know to treat Patient C with drug "b". Although not shown in FIG. 3, it is contemplated that Physician 3's use of the system necessarily results in Patient C's outcome/response to be input to the system to adapt the rankings of the treatments of the same molecular and clinical profile with drug "b".

Up to now cases such as those of FIG. 3 would have been reported in peer-reviewed medical journals, which take at least 6 months and can be searched in PUBMED, which has no proper search engine specifically for case studies since it is not searchable for complex medical parameters. The other approach, these experiences are reported is the collection of cases by "round emails" to fellow physicians to write a more comprehensive publications about the clinical experience with particular genetic target. Thirdly, the most common current method is to collect such experiences in clinical trials for years and published them in peer-reviewed medical journals. Newer approaches like Cancer LINQ collect medical information from EHR systems of different healthcare providers, analyze the data statistically centrally, compare the clinical results in different providers and then issue recommendations for future use of the same drug directly to physicians without publishing it to accelerate the learning cycle and to learn from each patient "story" not only from those treated in clinical trials. Cancer LINQ differs from the present approach in that Cancer LINQ requires collection of data and then centralized analysis. The present invention does not require full collection and focusses on individual patient-level data, including molecular diagnostics, genetic profiles, treatments and outcomes, and medical history at the user level. Accordingly, the present system automatically builds a shared knowledgebase by the users and uses direct inputs molecular diagnostic devices and other diagnostic methods. This present invention can be used to contribute to the Cancer LINQ by accelerating the collection of information and development of targeted treatment decisions, which are based on well standardized input data such generated by molecular diagnostic devices, to a larger medical knowledgebase, which include less structured parameters which have to be reviewed and analyzed manually.

### Therapy Ranking

The system can be applied to annotate molecular and other diagnostic parameters to any therapies. Data collected by the "use it share it" architecture can be analyzed by various statistical methods known by knowledgeable experts. As an example in case of one embodiment, the targeted therapy of cancer drugs are be ranked based on their response rate in previously treated patients similar to the present patient. Since molecular genetic alterations are analytical most reproducible and specific parameters and can be directly linked to the mechanism of action of targeted therapies, the ranking prefers the experience with patients with the same "driver" genetic alterations. In some exemplary cases, these are point mutation, copy number variations, translocations, but the system can also be based on any molecular diagnostic, proteomic, or metabolic analytical method.

If there is no experience yet with the exactly same alterations, the drugs are ranked based on the clinical results in patients with alterations in the same "driver" genes. If there is no experience yet with the exactly same genes, experience with "driver" genes in the same signal transduction pathway, therefore the same molecular targets, is considered. If there is no experience yet with the same targets, the ranking is based on the tumor type.

Most effective drugs in patients with the same: molecular profile/"driver gene" mutations > "driver" genes > "driver" pathway/targets > tumor type. For example, if there is experience with patients with the same genetic alteration, driver gene or target with the same tumor type, then the results of this combined database may be preferred, for example. If the clinical results in any of the later based experience outperform the previous category, the ranking of the better category is preferred.

Other parameters can be also used like the hereditary genetic profile, previous treatments, environmental and behavioral factors (e.g. smoking). But users can add any new parameters they want to collect information about and ask others to collect information and share it.

The patients may be treated first with the most likely effective treatment. For example, in case of primary resistance, they are treated with the second most likely effective treatment. In case of primary sensitivity and secondary resistance, new samples may be taken from the tumor for additional molecular profiling to explore the secondary genetic alterations, for example. Tests can be suggested by the system based on the experience with similar cases before. If a new biopsy is not possible, low frequency alterations in the previous sample and experience on most frequent secondary mutations may be considered.

### Calculation with Untested Confounding Factors

If there is information about the clinical response to any compound in a subset of patients further characterized by parameters, which are not known in the present patients, the probability that this parameter is present can be calculated into the ranking. The frequency of that parameter in the presence of the known parameters may be known from the annotated database/register of the system. For example, in case of known 90% response rate in the presence of a driver gene mutation that occurs in 10% of the given tumor type, the calculated response rate if the status of this driver gene is unknown is 9%. Another example is when the presence of a driver mutation predicts resistance but the status of this gene is unknown. The third example is the confounding effect of a second or multiple driver mutations.

If sufficient data is available in the unselected patients, then the direct experience based ranking is preferred.

In addition, the "test calculator," described further below, can be used to list genes, which should be additionally tested before the therapeutical decision is made if it is possible (sample and time is available).

### Test Calculator

Physicians only ask for any diagnostic test if they know its therapeutic relevance. There is also a need to select the most cost efficient diagnostic service for the specific clinical situation. In addition, when limited amount of tissue sample is available, it is vital to focus on the most relevant tests.

The "Test Calculator" can list the genes or other diagnostic parameters based on their relevance in the given patient. This is calculated by the experience and evidence based information about parameters, which can alter the ranking of drugs and the frequency of the given parameter in the presence of the already known parameters.

The "Test Calculator" can regard or disregards compounds in clinical development and can work together with the "trial calculator" to consider diagnostic tests related to compounds which are realistically available right now for the given patient. For example, for patients with very poor condition (poor performance status) who are not able to enter clinical trials and can be only treated with drugs with low side effects, and rapid action (called "Lazarus" response) diagnostic tests related to such drugs are considered only even if they target genetic alterations which have low frequency.

The "Test Calculator" can also list gene panels of diagnostics providers in order which contain the highest number of the important genes relevant for this patients but not redundant. Another way to rank the panels is to predict the chance of providing novel information, which can alter the treatment strategy. The Test Calculator may also provide a mechanism/portal, such as a dialog box or form, via which physicians may order tests identified by the test calculator as useful in the treatment and diagnosis of a particular patient based on the patient profile and information from the present system.

### Combination Therapies

In the presence of multiple drivers, combination therapies can provide solution. Combination therapies may be ranked the same way as mono-therapies based on the experience in patients with a certain combination of mutations.

### Development of anti-cancer treatment combination with the system, which can eliminate secondary resistance in cancer

The greatest challenge in targeted treatment is the emerging resistance due to the constant mutagenesis of the DNA. As the tumor grows more and more, subclones with additional driver mutations emerge which can be the source of relapse. The solution may be to use a combination therapy upfront against the expected secondary driver genes as soon as possible while the tumor burden is lower. The present system provides a mechanism to help to collect experience on the frequency of secondary driver mutations in a given tumor type, presence of primary driver mutation, and previous therapy. Experience with combination therapies in case of multiple driver mutations can be used to initiate combinations upfront, which give a chance to complete tumor eradiation and cure. For example, experience with these combinations will emerge and can lead to the optimal treatment of cancer. Based on the expected secondary driver mutations the best treatment known by the system against those drivers can be added to the combination therapy. Additional (third) mutations, which can cause resistance to the first and this second drug, can be also predicted by the system. The aim is to find treatments whose potential secondary resistance mutations do not overlap. The likelihood that in the same cancer cell two independent resistance mutations occur at the same time is very low, therefore the total elimination of cancer cells become theoretically achievable for cancer patients, which is a goal of the present system.

### Generation Of Scientific Evidence with the system

The present system also generates evidence based on the experience of the users to support or reject the hypothesis that a genetic alteration is a "driver" cancer gene or not, or if a drug target is positively or negatively associated with a driver gene or genes. For example, the observations of higher frequency of specific mutations in a certain tumor type, exclusivity with other known drivers, association with better or worse prognosis, and association with decreased or increased sensitivity to drugs targeting targets linked to the particular gene. The statistical proof of such associations can, for example, prove that a certain mutation or gene is a "driver" gene. This information can be used when the experience-based database/register has insufficient data about clinical responses to therapies in the presence of that mutation.

### Combination of Experience and Evidence Based Decision Support

The experience-based treatment calculations can start after the first user in theory, but as long as high case numbers and long follow up is not reached, evidence based decision support is more effective. Evidence may be generated by the system and can be added also from peer-reviewed publications by the users. In addition, if published clinical and preclinical evidence show better clinical response in a group of patients than the results achieved in patients in the Experience Database, the compound is ranked higher than the experience-based compound in the Clinical Experience Calculator.

### Evidence-Based Ranking Method of Targeted Anti-Cancer Therapies

This is a method to rank targeted therapies, which most likely inhibit the "driver" cancer genes or pathways activated by "driver" cancer genes in the given patient based on evidence.

The ranking starts with the ranking of driver genes by the "driver calculator." Next, drug targets (other than the driver themselves) are ranked based on the level of evidence indication (positive or negative) associated with the driver gene by the "target calculator." Next, drugs targeting the targets are ranked based on evidence and level of inhibition.

### Driver Calculator

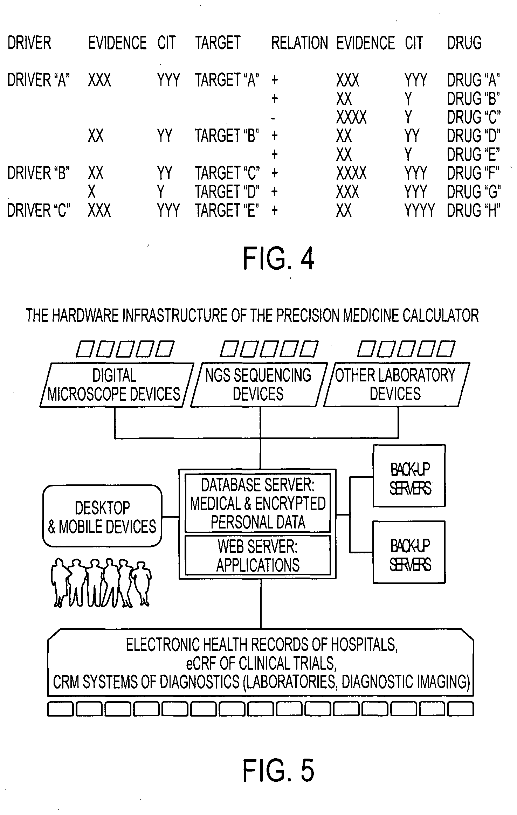

As illustrated in FIG. 4, in the first step genes with genetic variations are ranked based on the level of evidence, which indicate the likelihood of being "driver" (or deleterious) and not "passenger", non-functional alteration. The ranking is based on the frequency of that mutation in public databases like COSMIC, SNP and the database generated by the users of the present system. Other type of evidence is any published evidence, which indicate that a mutation is pathogenic or benign.

### Target Calculator

The driver genes themselves are often not pharmacologically targetable. Targets in the signal transduction pathways downstream of the drivers can be targeted alternatively. Other example for positive association is synthetic lethality. Negative association is also possible between driver and target genes. Examples are targets upstream of the driver genes. The positive or negative association can be documented by published evidence.

### Examples of published evidence codes:

Evidence for in vitro transformation of normal cells (loss of contact inhibition, immortalization)
Evidence for ligand or receptor independent signaling. (constitutive activation)
Evidence for exclusivity with other driver genes in the same signal transduction pathway (in cell lines).
Evidence for increased sensitivity to direct or indirect inhibitors in vitro.
Evidence for resistance to upstream or independent target inhibitors in vitro.
Evidence for increased tumor genesis or progression in transgenic mice.
Evidence for increased sensitivity to direct! indirect inhibitors in an animal model.
Evidence for resistance to upstream/independent target inhibitors in an animal model.
Examples of published and experience generated evidence:
   Evidence for exclusivity with other driver genes in the same signal transduction pathway (in human tumor samples).
   Evidence for increased frequencies in specific tumor types.
   Evidence for association with worse prognosis.
   Evidence for association with better prognosis.
   Evidence for increased sensitivity to direct or indirect inhibitors in clinical experience, (in a case study, retrospective analysis of a randomized trial, in a prospective phase Ia,b in a prospective phase II, in a prospective phase III trial).
   Evidence for resistance to upstream or independent target inhibitors in clinical experience (in a case study, retrospective analysis of a randomized trial, in a prospective phase Ia,b in a prospective phase II, in a prospective phase III trial).
   Evidence that this is a frequent germ line variant associated with increased risk of cancer (positive evidence).
   Evidence that this is a frequent germ line variant (benign polymorphism) which is not associated with increased risk of cancer (negative evidence).

Experience based database/register of the present system can also generate evidence based on the efficacy of targeted drugs in the presence of certain drivers. Although the efficacy of the drugs is also influenced by the strength of the driver and the biochemical efficacy of the drug as an inhibitor, the multiple experiences in cases of different genetic variations in the same gene and the experiences with multiple drugs add together to rank the driver-target association based on the most positive associations.

### Drug Calculator

Published evidence may be available about the pharmacological efficacy of targeted drugs against certain targets. In addition, the published and the experience based database/register of the present system can provide evidence as to which is the best drug against the target also in the presence of different drivers. The sum experience again can correct for the error introduced due to un-effective treatment experiences in the presence of false drivers.

### Signal Transduction Network Model

Since the shared experience based system provides information about driver-target relationships, this information can be used to develop correct cellular signal-transduction network models, which can store this information in a "neural network." This model can be used to *in silico* simulate the efficacy of future drugs, drug combination in cells with certain driver gene mutations and normal cells.

### Immunotherapy Calculator

Immunotherapies, e.g., cancer vaccines, are an extremely promising new approach to treat cancer. The hurdle with this approach is the low response rate in unselected patient populations similarly to targeted therapies. The present invention can help to annotate HLA genotypes of the patients and discover the relevant tumor antigens (epitopes) to clinical response to immunotherapies based on the molecular diagnostics of these biomarkers and clinical experience of the users. The ranking of the immunotherapies is based on the clinical experience of patients with the same Human Leukocyte Antigen (HLA )and antigen expression profile. In addition, evidence about associations between HLA genotypes and immune response to certain antigens generated by clinical observations can be used to evaluate the likelihood of response to novel immunotherapies.

### Personalized Management of Published Evidence and building an automatically shared evidence database by the Users

The current molecular information services and interpretation systems (e.g. Caris, N-of-One) collect evidence from public databases and rank the "strength" of the evidence based on the ranking system of "evidence based medicine" and the "quality" of the paper. This is usually done by dozens or hundreds of scientists supervised by dozens of editors. This means that a small group of people preselect evidence for the tenths of thousands professionals who have their own opinion and knowledge about publications in the 22 million publications in the PUBMED and hundreds of thousands of presentations at scientific conferences.

In the present system users can add any evidence to the system and decide if they want to share it with others. The user can review the evidence already in the system and make a personal decision whether to regard or disregard it. The users can also see how many users have used the evidence before which a type of citation or "like button".

The "strength" of the published evidence is measured by the impact factor of the biomedical journal where it was published and the frequency cited by the users of the system. The impact factor and the user's preference provide a community opinion how much they trust an evidence rather than the opinion of a group of scientists. The system may also have the ability to automatically rate and rank articles or collect information from users to rate and rank articles.

### Applications to Collect Standardized Information

The present system provides applications, which can be used by the users to get decision support, create report, ask for second opinion, but also they link data together in a standardized format, which helps to build an exact ontology of the experience.

### The Hardware Infrastructure of the System

An exemplary architecture is illustrated in FIG. 5. Users can access the system on their own personal computer, tablet or portable communication devices (smartphones) or other comparable interfaces. These devices provide the communication interface to provide input and receive output. The patients' personal data is only available for the user who has authorization. The medical data and the encrypted personal data are stored in a central cloud server. This server also has all the software and databases necessary for the system. This server may be backed-up in several places. The central database is also directly linked to diagnostic service providers and their diagnostic devices directly. For example, results of various tests may be directly input to the system, which incorporates the test data to the treatment ranking system, as discussed in further detail below.

### Direct Link to Laboratory Devices - "Digital Central Lab Concept"

The system of the present invention may include direct physical connection to several medical devices, which generate the input for the database. For precision medicine and shared experience based decision support, it is important to capture molecular data in a standardized way and to store all metadata of the data, especially metadata that may be important or essential to understanding and interpreting the data. These metadata information may include information about the methods (hardware and software) used to generate the data, and information about quality, negative and positive predictive value of the data. As can be appreciated by one of skill in the art, information or data that is received in a non-standardized way may be converted to the appropriate format for use in the present system, to include extraction of appropriate data and metadata and analogous conversion thereof.

### Direct Link to NGS

Presently, next generation sequencers (NGS) are important devices for precision medicine. These devices can sequence millions of DNA molecules in parallel cost efficiently. The machines of different manufacturers generate the same standardized output files, such as the FASTAQ. This file contains information about the nucleotide sequence the quality score of each nucleotide. This reflects the level of confidence if the information of a certain nucleotide is right or not. Next, this file is processed by different software. Users can modify the threshold for data quality. Next, different software with different algorithms are used to map the sequences to control sequences. The algorithms have different efficacy in their ability to find certain mutation types (1). These programs usually generate a text file (vcf), which contains the list of variations and the number of reads covering these variations. Next, the sequence variations have to be annotated to amino acid changes. The standard final output of the analysis is the list of genetic variations, which is documented in a molecular pathology report. As can be appreciated, the format used may vary, as long as the format can be converted to a format that can work with the present system.

Currently, in most cases pathology reports only report the fact that there is a genetic variation in the sample. All the information of the quality of the sequence, algorithm used to map the sequence, coverage etc. is not currently recorded. This means that clinical observations related to a certain genetic variation can be contradictory due to differences in the quality and methods used to generate the genetic information. This jeopardizes efforts of precision medicine to transform experience of each single patient with an extremely rare genetic variation into learning. This problem is usually handled in clinical trial by using a single central lab as the reference laboratory. All samples have to be sent to this lab from all centers, which slows down the process and cannot be used in routine clinical setting. The other disadvantage is that drugs are registered based on the results of a single lab which was may have been used as inclusion criteria or in the biomarker research. This means that the labs were not multi-centric, which is a significant risk of a catastrophic error. This was demonstrated by the erroneous analysis of EGFR gene in the biomarker analysis of the BR21 trial of erlotinib (Tarceva, Roche), which led to misleading information about the significance of genetic alterations of this gene in the selection patients who benefit from this drug (2).

The present system offers a solution for these problems by introducing the "digital central lab" concept. This is based on the realization that the files generated by the NGS machines of different vendors at different molecular pathology laboratories can be directly and automatically sent to the central server of the system and then processed by a standardized way, and all raw data can be stored centrally. The pathologist users can generate report online while oncologist users can make their decision on the bases of precise data and metadata, which is data about the quality and methods used for analysis. Clinical experience can be annotated this way to the precise information of the molecular genetic analysis. This can be used to more precisely clinically annotate genetic alterations and filter out erroneous data.

Further these quality and metadata information can refine the ranking of the different therapeutic options. For example, a low quality information on a clinically very significant driver gene mutation can be more important than a high quality information about a low significance, likely passenger variation. Such information can be coded in metadata so that the system properly takes into account the significance of the data in the input signal.

There are many cloud-based bioinformatics tools for NGS data processing for example mapping and annotation for professionals who process NGS data. This present system is different since here the raw sequencing data is associated with other biomedical data of the patient in the cloud and can be interpreted by the treating physician in context with other patient related information.

### Direct Link to Digital Microscopy

The other frequently used methods in molecular pathology are the immunohistochemistry (IHC) and fluorescent in situ hybridization (FISH).

Immunohistochemistry is used to stain proteins in histology sections and cytology smears to assess gene expression. Common examples are the estrogenic receptor and HER-2 receptor in breast cancer. The expression level is judged by the pathologist under the microscope, who ranks the intensity of staining (1-3+), estimates the percent of staining positive cells. Sometimes these values are multiplied by each other generate scores. The manual and subjective evaluation of these staining introduce a great ambiguity when we want to compare results done in different laboratories.

The other important method is FISH, which is used to visualize chromosomes and parts of chromosomes to evaluate the copy number and translocations of genes. When manually evaluated, the pathologist have to count the number of different fluorescent dots in many cells which is very time consuming and sometimes ambiguous for example in case of overlapping cells.

Recently, slide scanners that scan the slides in high resolution have become available. The digital image can be shared by the pathologist who can review the image remotely. The same automatized image analysis systems are developed for automated evaluation.

In this method the picture of the IHC and FISH stains can be scanned, manually or automatically, and sent to the central database and analyzed by standardized programs to generate uniform data with all details about the level of positivity and ratio of positive cells. Information can thus be encoded an input directly from the automated devices to the present system.

There are other medical devices without limitation whose raw data can be sent to the system, like real-time PCR or expression array scanners to any new devices. In such case, it is contemplated that the systems are capable of generating standardized information signals for input to the present system.

### Direct Link to EHR and Other Diagnostic Devices

The personal data, laboratory results, imaging results are stored also by existing electronic health record (EHR) systems and eCRF systems of clinical trials. These databases can communicate with the system. Alternatively, devices and computers of laboratory devices and imaging devices can directly linked to the system to provide the raw data.

### Workflow of the Molecular Treatment Calculation

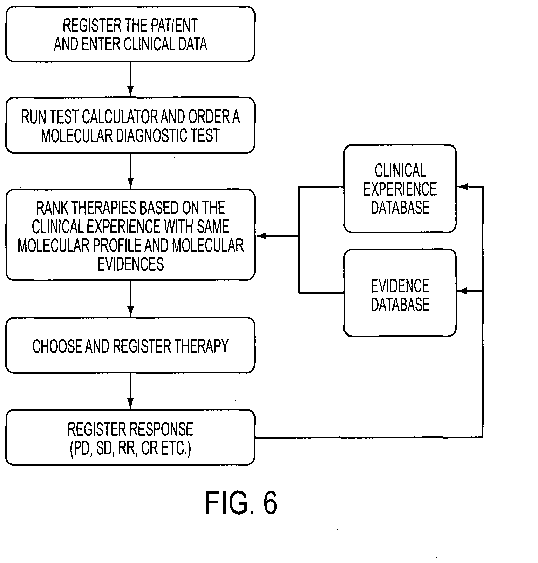

As illustrated in FIG. 6, an exemplary first step in the molecular treatment calculation is to register the patient and enter clinical data. The personal information can be filled out by the patient user, too. The patient user can send a request to a physician to be connected. If the physician fills out the information of a non-user patient, the physician either sends an automated email, which asks the patient to register and consent for sharing his/her biomedical data. If the patient is not computer friendly, the physician prints out a consent form and the patient to sign. The patients can consent to share their anonym medical data for the shared database to help medical decisions and research. The patient can consent for their physician contact them if there is a new clinical trial or treatment or diagnostic option for them. The patients can consent to share their personal data with the system to receive direct personalized information from the system. The physician can share the patient profile with another physician, surgeon, pathologist user etc. to consult the case anonymously. The patient can share his/her profile with multiple physicians. The clinical history is filled out by the treating physician, the molecular information can be filled out by the molecular pathologist or partner diagnostic lab. By using the Molecular Alteration Calculators by the molecular pathologist or by linking the system directly to the laboratory devices.

An exemplary second step is to run the "Test Calculator" and order a molecular diagnostic test. Based on the clinical profile and previous molecular diagnostic result, the test calculator can suggest single gene tests and diagnostic panels offered by diagnostic service providers. The user physician can order the molecular profiling. The system may facilitate ordering of any of the tests by contacting the test provider or generating a test order or otherwise providing a mechanism or portal for the physician to request the test.

An exemplary third step is to rank therapies with the system based on the clinical experience with same molecular profile and molecular evidence, as exemplified in Table 1 below.

**Table 1**

| DRUGS | Columns of Clinical Experience Values in this group of patients (same molecular profile, same driver, same target, same tumor type) Average Efficacy: PD. SD, PR, CR, PFS, OS Average Side. effects (grade) | Evidence Level Columns Based od published evidence and generated by the system (Driver, Target, Drug evidences) | Other information columns about the drug (e.g. price, availability, recommendations, registration, coverage by insurance |
|---|---|---|---|
| Compound "X" | X% | X | ...... |
| Compound "Y" | Y% | Y | ..... |
| Compound "Z" | Z% | Z | ...... |

Referring to the table above, the ranking of the therapies are both based by the clinical experience of the same group of patients (same molecular profile, same drivers genes, same tumor type) and evidence (published and system generated evidence calculators supporting the most likely driver, target and drug).

The treatment rank contains the name of the therapy, next in the same row the average response rate to this drug in patients treated before with the same molecular alteration, next it can be the average response rate of all patients treated before with alterations in the same gene, with the same target, the same tumor type or any other parameter. Next, it can be the evidence level, which support the driver, target targeted by the drug.

Referring to Table 1, the drugs (rows) can be re-ranked by any of the columns, to rank first the drugs with the best response rate (PR, partial response and CR, complete response) or diseased control (SD, stable disease included), PFS (progression free survival) etc. in any of the categories (same molecular alteration, target tumor type etc.) or by the highest evidence. The recommended ranking is based on the high response rate and highest evidence associated with the most specific category.

In case if there is only clinical experience with unselected patients, but the patient has a molecular profile that has a driver which is positively associated with the target or lacks potentially resistance causing alterations, the clinical experience data can be "corrected" to indicate an estimated potential clinical benefit to the particular patient based on the known alteration frequencies. If the clinical experience is only available in the presence of an alteration, which is not measured in the patient, the clinical benefit can be also calculated, based the frequencies of this alteration. The therapies can be ranked based these calculated potential clinical benefits.

In case of multiple driver mutations, the ranking is based on the clinical experience with the same combination. If this is not available, based on the clinical experiences with the alterations separately. The evidence for each driver are combined together, for example, added, in the evidence calculators. This can also adjust the sum of the evidence if one of the drivers is negatively associated with the target and its drug and lead to a change in the evidence-based ranking.

The professional user may choose any of the therapies based on the success rate, number of previous treatments, level of evidence and clinical situation. For example, if the clinical experience with the previously used drugs is poor, a new drug with high evidence can be tried. Also, if the clinical goal is to shrink the tumor to increase surgical resectability, then the objective response rate is relevant, and in other cases, a stable disease with low side effect profile is preferable.

A fourth exemplary step is choosing and registering the therapy. That is, the treatment decision is registered in the CRM system and the next visit is scheduled.

A fifth exemplary step is to register the response (PD, SD, RR, CR etc.). Upon next visit, the physician registers the outcome by clicking on response, SD, PD. The physician and the patient user can also register side effects (grade 1-3). The compliance of the user to register the drug used and the clinical response is ensured because when the tumor is progressing a new therapy has to be chosen using the system. At this point the user has to register previous therapies and responses because it is necessary for the test calculator and treatment calculator as well. Again the system does not ask users to enter data as an administrative requirement, but simply follows the "use it share it" principle.

A sixth exemplary step if for the system to automatically update experience and evidence-based databases.

### For example

= System adds the drug response of this case to molecular alteration/tumor type etc.-drug response clinical experience database
= System adds drug response of this case to drug calculator target-drug response clinical experience database
= System adds, "Drug response" to (driver-) target evidence calculator
= System adds this case "frequency evidence" to driver evidence calculator
= System adds "tumor type specific frequency evidence" to driver evidence calculator
= System adds "exclusive mutations evidence" to the driver evidence calculator
= System adds drug response to tumor-drug response clinical experience database
= System adds drug response to tumor type-drug response clinical experience database
= System adds drug response to parameter x-drug response clinical experience database

### Examples for useful applications, which can be used by the users, which also help the advancement of learning and shared knowledgebase:

### Online Digital Pathology Consultation

Digitalized pictures (uploaded manually or from the digital microscope) of histology can be shared with experts for second opinion online to ensure low variability of the information about the histology type. Image analysis systems can be used to standardize evaluation and to calculate tumor/normal cell ratio.

### Next Generation Sequencer (NGS) Mutation Calculator

Diagnostic labs, molecular pathologists, etc., can generate diagnostic report by entering exact data from sequencing runs. The data can be entered manually or from files generated by the NGS. Based on the tumor cell number, ratio, coverage, allele ratio, QC score, positive and negative predictive value of the status of a certain section of gene can be calculated. This value can be used later to correct the experience based database and to make decisions during driver gene ranking and therapy selection. The ratio of cells with the mutant allele can be calculated. Metadata about the type of sequencer, reagents used etc., may be stored too, and the metadata can be included in signals transmitted so that the system of the present invention can automatically read the signal information and adjust ranking. If diagnostic labs enter these data into the system they can use the molecular pathology wizard to print out their report if this is necessary.

### Immunohystochemistry and Fluorescent in Vitro Hybridization Calculator.

The expression level and ratio of positive cells by immunohistochemistry may be exactly recorded manually or be image analyzed. These possibilities existed in previous systems but the advantage of this feature in the present system is that this way the expression level of a protein will be exactly annotated to a clinical response of a drug. Likewise, the exact copy numbers of genes and ratio of cells with elevated copy number detected by FISH have to be entered into the database for clinical annotation. Automated calculation of immunohistochemistry scores, FISH scores, and molecular pathology report wizard, via the present system, can help the work of molecular pathologists and provide direct input to the present system.

### Online built-in CRM System for Physicians

It is contemplated that the experience based decision support system according to the present invention receives feedback about the clinical responses. Accordingly, the user experience and compliance can be enhanced by providing an online patient treatment timeline tool to physicians to easily keep record of appointments, diagnostic tests, treatments and their efficacy.

### "Cancerbook" of Users

Patients (healthy people, too) and physicians may be registered in the system with their personal profile and data. Patients can share their personal data and medical records in the system with any doctors registered in the system.

Patients can also share information with other patients. Patients can consent to share their anonym medical information and also share their personal information. ("Fight cancer by sharing your story").

Physicians can share their experiences, evidence they uploaded to selected users or all users. They can share the number of evidence they uploaded and their success rate. The treatment decisions in certain situations of opinion leaders can be tracked if they consent.

### Digital Tumor Board

Physicians can also share their patients data with the consent of patients to other doctors to initiate consultation, second opinion. Special consultations can be initiated with special applications to ask for online second opinion from pathologists, surgeons, radiologists. This further standardizes treatment decisions and helps further specialization of physician while enabling cooperation.

### Personal Clinical Trial Calculator

Users (principal investigators of clinical trials or clinical research organizations) of the system can upload inclusion criteria of clinical trials and decide if they want share this information with others to receive patients from other physicians in the same country or even from abroad. This is different from other known clinical trial databases, which are not maintained by the clinical investigators themselves.

Further advantage of the trial calculator embedded of the present system is that the clinical and molecular parameters of the patient are known for the system therefore only trials which are really relevant will be listed, the user do not have to manually check all inclusion and exclusion criteria.

If a physician wishes to refer a patient to a trial, the clinical and molecular profile can be shared with the PIs of the trials for referral and/or further prescreen. The trial calculator also works together with the therapy ranking algorithms to help in the selection of trials that test compounds that are most likely effective in the given patient. The trial calculator also works together with the test calculator to suggest which genes should be tested based on the clinically matching trial options the patient currently has.

The trial calculator will be able to work "backwards," too. The user will be able to search for matching patients in the database. Physicians can identify the patients and patients can receive an alert if they match a search. Users can also see how many matching patients are at which physician's database and can contact the physician and suggest to refer into this trial.

The unique feature of this patient database is that the patients have given their consent to the users of the system to search their biomedical data and also consented to be contacted if relevant clinical trials emerge.

The present system also may allow a patient and/or physician to identify new or ongoing trial for patient participation, as well as allows for organization planning or conducting a clinical trial to identify patients via the system (whether information has been entered by the patient or the physician) for participation in a clinical trial well matched to the patient profile, for example, by tumor type, histology and/or molecular profile.

### Treatment Strategy Calculator

The combination of information provided by the MTC, guidelines of FDA and professional organizations (e.g. CancerLINQ) and the inclusion-exclusion criteria of clinical trial can help generate a road-map of the treatment strategy which maximizes the treatment opportunities for the patient. For example if a second line clinical trial is available for patients with a specific molecular alteration, but the inclusion criteria indicates a certain first line treatment, the physician user can think ahead and choose the right first line therapy to secure the opportunity for the patient to participate in that trial. The aim of the treatment strategy calculator is to always think one step ahead.

### Marketplace and online built-in CRM for Diagnostic and Interpretation Services

Since the personal expert system gives back the freedom and power to physicians to make their own decisions on treatment recommendations, diagnostic tests like molecular profiling of the tumor can be requested by the physician and ordered by the patient using the system.

The decision of which tests to order will be supported by the "test calculator." The results then are integrated into the profile of the patient. The results are then interpreted by the physician with the system.

For partner diagnostic services, the system will be provide online payment, online ordering, reporting and CRM system to follow up requests and process, notification of diagnostic steps and estimated time until results.

There are also public and private interpretation services whose database or service can be ordered from the platform using the molecular profile of the patient tested by independent diagnostic companies. For example, there are *in silico* predictors of function relevance of genetic variations. The resulting data can be directly input to the present system.

The advantage for the system to link these service providers directly into the system is to obtain standardized, quality controlled biomedical data.

### Experience Generated From in Vitro Drug Sensitivity

In vitro survival cultures and xenografts in mice (avatars) are established from more and more tumor samples. In this models a limited number (10-20) of treatments (and treatment combinations) can be tested. This expert system can be used to select the top 10 therapies and next these can be tested in the patients tumor cell cultures in vitro. The best drug can be suggested then to the patients. The results of the simultaneously tested 10 drugs can be fed back to the system to accelerate evidence database building using this *in vitro* data.

### Engagement of Patients

Patient users will also register to the system and will be asked to create their personal profile, e.g. their "cancerbook." They can use the system to look for physicians, consultation with specialist and find other patients with similar profile. Homepages for patient groups already exists, for example "PatientsLikeMe" where patients can meet other patients with the same disease to share their experiences, but these online communities are not linked directly to medical information, which can be provided only by physicians and medical devices. Therefore, these systems cannot "learn" automatically which the best therapy is and support medical decisions instantly.

Patients can also choose to receive relevant timely new information about their conditions, novel therapies or clinical trials matching their molecular or clinical profile. Patients will be advised to visit their physician to discuss such possibilities. Physicians will be informed about the same possibilities in parallel. Similarly, physicians may be alerted, for example, when certain criteria are met, which may be preselected by the physician, about newly available, reimbursable or registered drugs, tests, trials, etc. The criteria may be patient specific, tumor specific, profile specific or some combination thereof or based on other selected information as may be appreciated by the physician. The goal is to help cooperation between patients and doctors. In summary, the system can help patients to find the best specialist, find cure as soon as it is found in similar cases in the world, to share their story with the world for cancer research.

### Side Effect Calculator for Patients and Therapy Ranking Based on Experience with Side Effects

Registered patients can track the side effects of treatment and get help if necessary. Meanwhile, this information is linked to the experience-based therapy ranking, which can estimate the likelihood of side effects for new patients. This can be the basis of quality life based ranking. Side-effect information may be provided by patients, physicians, trial providers or any users, as appropriate.

### Engagement of Healthy People

People who are healthy can also register to start prevention and contribution to the fight against cancer. After registration they can calculate their risks for examples for certain cancers. Such calculators already exist. The novel approach here is the instant data sharing between the registered users and accumulation of experience, which in turn is used by the calculators. Additional value is the possibility of combination of the database with the expert system for patients and physicians. Thirdly, there is a direct link provided by the system to physicians and healthcare providers who can help in prevention, for example screening with colonoscopy or CT. The results are immediately fed back to the shared experience database if cancer is detected and the patient visit a registered physician who start to calculate the treatment.

For example, if a registered user calculates BMI in the system the user enters weight and height. Next, BMI is used to calculate how that BMI affects the risk to different cancers. This is calculated based the frequency of those cancers in users with the same BMI who used the same system. This is possible due to the data sharing architecture of the system. If a registered user enters data, it is linked to the user anonym virtual identity and shared with the central database. Therefore, if this patient is diagnosed with cancer, the BMI is annotated to a clinical event.

### Annotation of Hereditary Genetic Variations

A special and very important goal is the annotation of hereditary genetic variants to clinical relevance, for example cancer risk. In this system patients can enter can their genetic information and family history and run a calculation if there is a need to visit a genetic counselor or cancer prevention specialist. Here again the genetic information is linked to the virtual, anonym identity and clinical experience is collected, shared and annotated to clinical relevance automatically

### Advantage of an Integrated System for Patients and Physicians

The engagement of the general public into the data collection against cancer can solve the problem to identify the clinical significance of low penetrance environmental factors (like diet) and genetic variations in large number of patients and creates a never ending epidemiology study. The integration of the system with the medical system ensures the professional curating of the data.

The anamnesis of the patient before the onset of a disease can greatly help diagnosis and therapy decisions. For example, people with smoking history and family history tend to have different mutations, which can influence the "test calculator" and the therapy ranking. For example, a certain anamnesis is linked to the high prevalence of a genetic variation, which is in turn predictive of sensitivity to a drug, the therapy ranking can suggest the right treatment without actually testing.

The pre-recorded information by the patients can reduce the cost of administration if it can be linked to other health records.

### Competition/Coopetition

This is the first interpretation system, which is directly available to physicians to clinically interpret the molecular genetic profiles themselves to support treatment decisions independently form diagnostic service providers.

Traditionally, the physicians have interpreted laboratory tests, including molecular pathology tests. But as multiple gene testing were introduced, diagnostic companies like Caris who provide multiple gene testing started to provide the molecular interpretation of their own tests to the physicians. Foundation Medicine is also providing molecular interpretation service for its own diagnostic tests. N-of-One provides interpretation service to diagnostic service provides, which in turn provide this to physicians. Molecular health is interpretation provider, but only for users who order the diagnostic test from them.

The present new independent interpretation tool can give back the power and responsibility of doctors to make treatment recommendation based on the latest scientific evidence, and their own and colleagues' experience. In fact, diagnostic services and current evidence database driven interpretation services are not competitors to the present system. Instead, they provide the need and input to the present system. Users can upload the results of molecular profile analysis of these companies. User can select the given panel and type in only the variations, while other genes will be by default wild type for rapid data entry. Interpretation services can provide recommendations when experience based data is not sufficient.

Cancer LINQ (Learning Information Network for Quality) of ASCO aims to develop "rapid-learning health care" system. This is a health information technology (HIT) solution to link various electronic health record (EHR) systems. The data is then analyzed centrally to constantly ("real-time") assess compliance with performance measures of all healthcare providers. Cancer LINQ vision is also to learn from each cancer patient, not only from the 3% who participate in clinical trials but also from the 97% of patients who do not participate in trials. Therefore they also analyze the input data and clinical responses to therapies to generate new hypothesis for clinical trials and to update guidelines. Their vision is that if all oncologists use the same EHR system ASCO and regulatory agencies can update guidelines electronically, which will reach all doctors in "real-time." When they enter the clinical data of a given patient a pop-up window comes up, which tells the doctor about the actual recommendation how to treat the patient. The present invention is different from this system, since it directly links databases of users and instantly update treatment ranking as data of a new patient is entered. In other words, the present system is an "automated self learning" not just a "rapid learning" system. This system can also combine evidence-based and experience-based decision support.

The two systems can cooperate with each-other since the present invention is personal decision support system which is used to make individual decisions, and works only with exact parameters, Cancer LINQ generates guidelines how to treat group of patients after central analysis of the data and can also use less exact parameters since longer human curation is involved in the analysis.

Present method can be an interpretation solution, patient data and biomedical parameters of patients treated by the present system will enter the Cancer LINQ database either by the parallel use of this system and the local EHR or by developing an IT interface which feeds data into Cancer LINQ. The present system can integrate Cancer LINQ "real-life" guidelines into its decision support process and EHR/CRM.

### Value for Healthcare Industry/ Business Model

The present system creates unique value for many players of the healthcare industry, which allows the development of several innovative business models. The ultimate customers and users are the patients and physicians. However, several industry players can find commercial value, which can allow free access for patients and physicians.

Some examples without limitations: the present system can revolutionize drug discovery and biomarker research. First, current clinical trials targeting a subset of patients will accelerate due the large database of patients with known molecular profiles who have consented to be contacted, e.g., when a clinical trial is looking for a patient having a particular profile. Pharmaceutical companies, CROs who look for patients with rare genetic alterations into their clinical trials are ready to pay for the pre-screening and referral. Future clinical trials can be initiated based on the information in which oncology center how many matching patients are available. The present system allows for a search to identify and quantify cases meeting the criteria for the trial.

Repositioning of drugs already in clinical use can be initiated based on the electronic case report records of the system without any further clinical trials in orphan indications. This will help to reduce the clinical development and registrations costs of pharmaceutical companies, which may in turn will lead to the decrease of drug prices.

Existing label indications can be narrowed down into more specific indications by novel "biomarkers" and re-registered based on the information of the system, which increases cost efficacy for payers and reducing cost for pharmaceutical companies in case of outcome based financing.

The expert system helps the physicians to "remember" at which indications a drug should be used. This is extremely useful for orphan indications of rare subset of cancers with rare molecular alterations. Physicians can get instant and targeted news about novel drugs. Physicians who have patients in their database who could benefit from the novel therapies are alerted. For these patients the therapy ranking has to be repeated.

These services can greatly reduce the sales and medical marketing cost of targeted, niche-buster drugs. This is a very important feature of the system since in rare indications (one patient in 1-2 months) it is very difficult to educate physicians by medical representatives.

The test calculator can increase the cost efficacy of diagnostic test for payers. The test calculator and the available interpretation service on the other hand is a great marketing tool of diagnostic services, diagnostic reagent and equipment manufacturers.

The online payment, ordering, reporting and integrated CRM system for diagnostic and interpretation services help even novel start up biomarker companies, to set up their services, and access the whole market instantly.

The integrated expert system also make sure that the information provided by the test is used in the decision making process. This is also a very important feature of the system, which increases quality assurance. In particular, somatic genetic variations can change during the course of the disease, which may lead to forgotten molecular diagnostic result.

The platform can also help third party digital health applications to develop compatible applications based on already available features and reach patients and physicians rapidly.

### State of the Art in Cancer Research

The recent fast track clinical development and market authorization of crizotinib for ALK translocation positive non-small cell lung cancer (NSCLC) is an excellent example for integration of molecular diagnostics into anticancer drug discovery. In this case the clinical development focused on the 4-7% of NSCLC. The 4-7% seems to be a small fraction of lung cancer cases but actually this is a "hill" in the molecular landscape of lung cancer. Crizotinib also inhibits ROS1. ROS1 translocation occurs in 1,2-2,6% of cases. ROS1 is a "knoll" in comparison to the hill of ALK. Crizotinib is already routinely used off label in ROS1 positive lung cancers and striking clinical responses have been reported in case studies. Similar striking clinical results have been observed previously in case of BCR/ABL translocation, EGFR activating mutations and BRAF mutations. The common feature of these targets now all considered "driver" cancer genes. Driver genes therefore provide an attractive target for anticancer drug discovery. But, in the genomic landscape of cancer, there are only a couple of "hills" with the average eight of 5%, where drug discovery strategy with clinical trials focusing on pre-selected population can be successful.

Even if this drug discovery approach is used, pharma companies are reluctant to conduct the same registration trial for these small subpopulations of each tumor types and leave the decision to the treating physician to use the drug off label in another tumor type. In addition, in more than half of the cancer patients we find rare "driver" mutations, or specific combinations of mutations, which represent extremely rare molecular profiles.

The other problem is the rapid development of drug resistance due to activation of feed-back loops, selection of resistant mutants or complete driver pathway switch. Combination therapies are needed to overcome resistance and achieve final cure of cancer. In this review, we summarize, why and how we have to integrate anticancer drug discovery into the clinical practice in order to find effective targeted treatment for this large population of patients with rare driver mutations.

In our review in Nature Reviews Drug Discovery, we suggested the co-development of companion diagnostic test and the targeted compound (1). The concept was that this way the clinical trials could focus on the subset of patients who will most likely respond, which although reduces the potential market size but also decreases the cost of the trial and accelerates the approval. Many pharmaceutical companies have followed the same concept. For example, crizotinib (Pfizer) was tested only in patients with NSCLC positive for ALK translocation (2). The BRAF inhibitor vemurafenib (Roche) was tried and approved for BRAF mutant melanomas (3).

In these clinical trials the biomarker analysis was performed with a clinical trial assay typically in a central laboratory. The exact method and its vendor used for the clinical trial indicated in the label of the drug. The FDA regulates companion diagnostics as medical devices. Theoretically, the drug is indicated for the treatment of patients whose tumor has been tested with the particular test. Examples are for the above-mentioned examples: Vysis ALK brake-apart probe (Abott) for crizotinib and COBAS 4800 (Roche) BRAF test for vemurafenib. The argument is that the safety and efficacy of the drug is only proven for patients selected with these tests.

Pharmaceutical companies can also use these tests as marketing tool. If the particular test is used for the diagnosis the particular drug has to be used which is linked to the drug and not the competitor drug for the same target. Pharmaceutical companies like Roche traditionally have diagnostics products others have acquired diagnostic companies to co-develop and co-market the drug and its companion diagnostics. Linking the drug and the companion test was a new strategy for Roche too since before in case of trastuzumab molecular diagnostics for HER-2 overexpression and copy number gain analysis were marketed independently and the pharma and diagnostic branches did not even cooperate. It could happen that the pharma branch recommended a cheaper competitor of Roche diagnostics to help molecular pathology labs complete the test necessary to sell the drug.

In countries where the financing of molecular diagnostics was not solved by the national health insurance pharma companies financially contributed to the testing by directly paying to molecular pathology laboratories or by providing kits. This was the case of KRAS testing and EGFR immunohistochemistry for cetuximab by Merck and EGFR mutation analysis for gefitinib by AstraZeneca.

### Clouds on the Horizon of Companion Diagnostics

The golden age of one drug one test companion diagnostics is closing to its end. Now we have many competitor drugs for the same target. Merck and AstraZeneca ceased or decreased support of companion diagnostics since erlotinib by Roche and panitumumab by Amgen are registered for the same first line indication for lung cancer and colon cancer patients. The reason is that they cannot control which drug the physician will subscribe for the same results.

Now pharma companies try to use the companion diagnostics to establish their niche. For example, in case of vemurafenib the V600E point mutation of BRAF has be detected in melanoma with Roche own diagnostic product and equipment the Cobas 4800. Dabrafenib and trametinib (GlaxoSmithKline, GSK) is prescribed also for V600E (and V600K) mutant melanomas but this case the companion diagnostic device is the THxID^{™} BRAF Kit (bioMerieux Inc.) (Source: FDA homepage). Therefore, if the pathology laboratory of a oncology center decides to buy a COBAS real-time PCR machine and uses the COBAS Kit for BRAF testing and detects a BRAF mutation the physician cannot subscribe dabrafenib only if the BRAF test is repeated by the ThxID kit. This is a non-sense in a real-life setting from analytical point of you and since the tissue sample and time is limited.

The same situation is true for the two EGFR MABs cetuximab and panitumumab (cetuximab is linked to Theracreen KRAS test), and the two EGFR inhibitor TKI gefitinb and erlotinib (erlotinib is linked to COBAS 4800). Standardization and quality assurance is indeed is extremely important in case of companion diagnostics. However, ones who work in labs know that 90% of the serious errors occur in the pre-analytical phase, which are not controlled by the closed IVD marked diagnostic kits. For example, in pathologies tissue samples are processed in an environment not designed to be molecular grade clean. Simply the tissue samples from different patients are sliced on the same table and then fixed and embedded in machines in the same liquid among dozens of samples. The quality of formalin and time of fixation can vary greatly and microtome used to make the sections is not always cleaned properly. In the molecular lab it is also very easy to cross-contaminate samples before the standardized IVD companion diagnostic device used. This is why it so important to regulate the laboratories. The best practice is the external quality assurance ring trial for example organized by the European Association of Pathologist for KRAS testing. In this ring trial sections of FFPE blocks are circulated, therefore the labs have to start from the isolation of DNA and can use any method they buy or developed by them (LDT test) to detect mutations. The lab passes the test if in 90% of cases the result is concordant with the consensus results.

### Should we Find Patients for a Drug or Find the Best Drug for the Patient?

The other dilemma of companion diagnostics is that many targeted drugs can be registered for different targets for the same patient. The first dilemma is analytical. For example, it is expected that imatinib will be registered for c-KIT mutant (8%) melanomas (4) and there are currently clinical trials for NRAS and HER-4 mutant melanomas. In case of melanomas the surgically removed tumor is quite small and due to well-known heterogeneity fresh biopsy is recommended for metastasis. The amount of DNA extracted from these samples is very limited, usually is enough for one test. Each target gene segment is present in each cell in two copies (in case of euploidity). If we use just one primer pair to amplify one target segment like the region of the V600E mutation in BRAF we waste all other DNA in the sample. If we do a multiplex PCR and add the primers for c-KIT we can use the same sample to detect also c-KIT mutation. The question in the lab boils down to the dilemma whether to use the sample for a companion diagnostic test of a particular drug risking that if it is negative the patient losses the opportunity to get targeted treatment. In other words: should we add the KIT primers or not. Even if the sample is larger, running two independent tests is always more expensive.

The other dilemma is clinical. For example, in case of non-small cell lung cancer the EGFR inhibitor erlotinib is registered for the whole unselected population based on the registration trial BR21 that increased the median survival from 4.7 to 6.7 months (5).

This includes the subpopulation of ALK translocated 5% where the driver oncogene is not the EGFR. The 1-year overall survival in this population in case of ALK inhibitor crizotinib treatment is 70% (!) versus 12% with other treatments (6). In these patient the response rate to erlotinib is 0% (7).

The ALK FISH test is not a companion diagnostic device of erlotinib. If a physician wants to prescribe erlotinib for a lung cancer patient no molecular diagnostic test is required. Erlotinib can be used without knowing if the patient is ALK positive or not.

If we ask the right question in the molecular age, what is broken in this tumor, what is the driver oncogene, what could be the best treatment for this patient, the answer in case of ALK translocation would an ALK inhibitor for sure. But if we follow the traditional evidence based medicine logic, erlotinib is proven to be effective over placebo in the unselected population of non-small cell lung cancer, therefore can be given without testing for ALK.

The other most recent example is the addition of NRAS testing to the mandatory companion diagnostic test of panitumumab. Panimutumab is an anti-EGFR monoclonal antibody originally registered for KRAS wild type advanced colorectal cancer patients. KRAS is mutant in 35-40% of colorectal cancers. NRAS is a homologue gene of KRAS in the RAS family produced by evolutionary gene duplication. Tumor cell biologists have shown long time ago that NRAS have hot spot mutations on the same sites and have the same biological relevance as KRAS. Still, we had to wait for evidence based medicine proof to add the testing of NRAS - which occurs in 5-10% - to KRAS and now we to talk about "RAS" test, which means a two gene panel (8).

Cetuximab is also an anti-EGFR originally registered for all colorectal cancer and later restricted for KRAS wild type cancers like in case of panitumumab. Between the May of 2013 and December of 2013, NRAS testing was only mandatory for panitumumab not for cetuximab. In this period, physicians who followed the rule of evidence-based medicine chose panitumumab for KRAS/NRAS double wild type and cetuximab for NRAS mutant colorectal cancer patients. From a molecular evidence point of view the use of cetuximab seems non-sense in an NRAS mutant patient, but from the evidence based medicine and regulatory point of view it was acceptable or even expected. In addition, oncologists who considered cetuximab in this period only asked for KRAS testing since NRAS testing was not "clinically relevant." This is the logic when we try to answer if we can use a particular drug based on the label or not. Today, NRAS testing is also mandatory for cetuximab as it was expected as a class effect.

The addition of NRAS to KRAS also affects the use of bevacizumab. Bevacizumab is an angiogenesis inhibitor whose efficacy is independent from the RAS mutations. The PFS and OS results in the KRAS wild type 60% of patients was very similar with panitumumab, cetuximab and bevacizumab therefore physicians decided which targeted drugs to use in KRAS wild type patients based on the side effects and other clinical considerations. Since now we know the EGFR inhibitors do not work in NRAS mutant we can further narrow the target population to around 50% KRAS/NRAS double wild type. By simple mathematical logic in this smaller population EGFR inhibitors must over perform bavacizumab since responder are now enriched. Luckily, this has been already proven in clinical trials (9). Now the dilemma is that RAS testing is not a companion diagnostic device of bevacizumab, therefore it is not mandatory to test RAS if the physician plans to prescribe bevacizumab. But if the question is which the best targeted treatment for the patient RAS testing should be performed.

### Evidence Based Medicine Proofs for the Concept of Molecular Evidence Based Medicine

The striking clinical activity of some already registered targeted therapies in selected patient populations is well known. The first breakthrough was the success of imatinib in BCR/ABL+ CML (10), and the effectively of EGFR-TKI (gefitinib, erlotinib, afatinib) in EGFR mutant metastatic NSCLC where the respond rates are between 70-100% and the median survival is close to three years (3,4). Most recent examples are the 81% response rate of the BRAF inhibitor vemurafenib in BRAF mutant melanomas and 90% disease control rate of crizotinib in ALK translocated NSCLC (2,3).

However, there are around 460 cancer genes and only around 30 targeted drugs in clinical use. Fortunately, there are over 200 targeted drugs in clinical development. Trials of targeted drugs often focus on patients with a specific molecular profile already in Phase I trials.

The first large clinical trial, which provided proof for clinical benefit of referring patients to even to phase I clinical trials or treat based on molecular profile was IMPACT (Initiative for Molecular Profiling and Advanced Cancer Therapy) in the University of Texas MD Anderson Cancer Center, USA (13-15).

The trial was started in 2007 and was published in 2012. During the 4,5 years 2,282 patients with advanced cancer of any tumor type were screened and in the tumors 12 genes were analysed with sequencing, immunohistochemistry and fluorescent in situ hybridization (FISH).

In 52,2% of patients, the molecular screening detected one or more mutations. Frequency of mutation in different tumor types were the following: melanoma (73%), thyroid cancer (56%), colorectal (51%), endometrial cancer (43%), lung (41%), pancreatic (41%), breast (32%), other gynaecology (31%), genitourinary (29%), other gastrointestinal (25%), ovarian (21%), head and neck (21%), sarcoma (11%), renal cell (9%). 440 patients (19,3%) were enrolled into Phase I trials based on the molecular profile and 442 into molecularly not matched trials. In patients who had one mutation, and were enrolled into a clinical trial based on the molecular aberration, the response rate was significantly higher (39% versus 15% P<0,0001), the PFS was also significantly better (4,9 months versus 2,2 months, P<0,0001), and the median survival was also significantly longer (11,2 months versus 8,6 months, P<0,006).

In case of molecular profile based Phase I trials, the patient had significantly better response to the experimental drugs in Phase I trial than to the previous registered chemotherapy protocol they received (TTF 4,0 versus 3,1 months, P<0,0008). In contrary, in unmatched trials patients had worse response in the trials (2,0 versus 3,2 months, P<0,0001).

In conclusion this study proves that even based on a molecular profile including only 12 genes, 19% of patients can be enrolled in matched Phase I trials, and these patients benefit from the molecular analysis. This trial in MD Anderson center only involved heavily pre-treated patients with an average of 3 lines of previous therapy, therefore the performance status of these patients was most probably were poor, further, these patients did not have the opportunity to enter molecular profile matched clinical trial open for patients with less prior therapies. Therefore, both the success rate, both the clinical benefit can be greater if the molecular profile analysis is performed in an earlier phase of patient therapy.

In Massachusetts General Hospital Cancer Center (Harvard Medical School, Boston) similar results were reported in NSCLC patients profiled with the mutation analysis of 15 genes (16). They reported mutations in 51% of patients. 8% of the patients had EGFR mutations and received EGFR inhibitor therapy, in addition, 14% of patients received treatment based on the mutation found in the tumor in clinical trials during the one year's follow up time. The authors noted that it was expected that more patients would receive treatment in matched clinical trials as they progress on current chemotherapy. The conclusion of the authors was that molecular profiling should be part of the routine clinical practice

In the Integrated Molecular Profiling in Advanced Cancers Trial (IMPACT) at the Princess Margaret Cancer Centre (PMCC), Toronto, Canada (17) advanced breast, colorectal (CRC), non-small cell lung (NSCLC), ovarian cancers and other solid tumors were analyzed with the Sequenom's panel (23 genes, 280 mutations). The 23 genes panel detected mutation in 137/349 (39%) pts, including 24/79 (30%) breast, 40/80 (50%) CRC, 54/88 (61%) NSCLC, 17/78 (22%) ovarian, and 2/24 (8%) other cancers.

After a median follow up of 5.0 months, 31/137 (23%) pts with mutations have been treated with targeted therapies. Clinical data was available in about 21 patients treated in matched clinical trials. 6 patients (29%) had objective response, 11 patients had tumor shrinking between 0-30% and 4 patients SD or less than 10% progression.

In the MOSCATO-01 (Institute Gustave Roussy, Villejuif, France) (18) a 50 genes panel (Ion Ampliseq Cancer Hotspot Panel, Life Technologies) was sequenced and CGH were used for copy number variation analysis in solid tumors (lung, head and neck, bladder, prostate, ovarian, breast, colorectal, oesophagus, gastric, pancreas, uterus, biliary track, renal, CUP, and other rare tumors). In this trial patients were also heavily pre-treated with the median of three lines of therapy, but with ECOG 1. 129 patients were screened. In 53 pts (47%) an actionable target was identified and 33 pts (29% of all patients) were treated with a matched targeted therapy, mainly in clinical trials. In these patients the clinical response to targeted therapy was 7 PR (21%), 16 SD (48%) and 7 PD (21%). Three pts (12%) were not evaluable. This is a much better result than the average 5-10% response rate observed in clinical trials (19). In addition, PFS ratio in comparison to previous chemotherapy was >1.3 among 9 out of 19 evaluable pts (47%).

In conclusion, a cancer panel of 50 genes provides actionable targets for at least 75% of solid tumor patients. Published data indicates that at least 30% of patients are treated with targeted therapy as a consequence of molecular profiling. Patients treated in matched clinical trials have significantly higher chance to benefit from the treatment than patients treated without clinical profiling.

### "On Target off Label " Treatments in Clinical Practice

Clinical oncologists more and more often use off label targeted drugs based on the molecular profile of the tumor. In these cases, the targeted drug is already registered for another type and the biomarkers or molecular profile positively associated with efficacy has been established. If the same the same molecular profile is found in another tumor type, the administration of the targeted drugs "on target" and "off -registered - tumor type".

One example is the successful off label use vemurafenib in BRAF mutant lung cancer cases (20,21). Vemurafenib is registered for the treatment of BRAF mutant melanomas where the frequency of BRAF mutations if 40% (COSMIC). The frequency of BRAF mutation in lung cancer is 5% (COSMIC).

The other situation when the targeted drugs used in a tumor type for the drug is registered but in a presence of a target which not the registered target of the drug. One example is the successful off label crizotinib treatment of c-MET amplified non-small cell lung cancer patients published independent case studies (22,23). The rational of these treatments was that although crizotinib is registered as an ALK inhibitor for ALK positive lung cancer, crizotinib is also a c-MET inhibitor (24).

### The Last Frontier: Tackling Resistance with Combination Therapies

Targeting a single driver pathway with monotherapy is not the final solution. Drug resistance develops very rapidly in most cases. All cancer driver genes can be mutated in any type of cancer and number of drivers varies between 2-8 in each cancer (25). Tumors with multiple oncogenic and tumor suppressor drivers show primary resistance to any monotherapy. The simplest situation when we have only one oncogenic driver and one tumor suppressor gene driver. For example, in case of activating mutations of EGFR, which exclude the primary presence of any other oncogenic driver, only mutations of the p53 pathway (26). The high addiction to EGFR in these tumors is reflected in high percentage tumor response and also by high percentage of secondary resistance mutations in the same gene (27). However, even in this tumors pathway switch to other driver genes like c-MET, ALK or BRAF do occur eventually (28).

Much faster resistance mechanism is due to activation of feed-back mechanisms. A recent example is resistance of BRAF mutant colon carcinomas to BRAF inhibition (29). It was discovered that in BRAF mutant colon carcinoma cells BRAF inhibition activates EGFR via the inhibition of the physiological negative feedback loop, which is activated by BRAF/MEK pathway. In melanoma cells EGFR is not expressed therefore BRAF inhibition can be effective.

The silver line in the clouds is that the resistance mechanism, show a pattern and seem to be predictable. This support the hope that combination therapies can prevent such resistance. For example, combination of EGFR and BRAF inhibitors are synergistic in colon carcinoma cells (29). In our recent work, we showed rapid activation of HER-3 in response to AXL inhibition, which could be overcome with combinations of HER-3 and AXL inhibitors (30).

The conclusion is not that molecular profile base targeting tumors is not the future, in the opposite; this means that we always have to know the whole molecular profile.

The question still remains if secondary resistance mechanisms due to the "natural evolution" of the tumor genome under selection pressure can be ever beaten. If we want to answer this question we have to use the experience of population genetics of evolution. In an interesting analysis of a group of mathematicians used the model of population genetics to understand the drug resistance of tumor cells (31). They found in fact that - if we calculate with the known mutation rate - by the time a tumor is detected, there is a high chance for the presence of resistant clones in the tumor burden. After initial response due to the sensitivity of the majority of cells these clone grow up and become detectable. By the time this second clone becomes detectable, new sub-clones with new mutations develop. Therefore, sequential monotherapies cannot even by theory cure cancer. The up-note is that if we combine therapies, which cannot be prevented with a single gene mutation, only with combination of mutations, there is a great chance for even a cure.

### EXAMPLES

### COLLECTIVE CLINICAL EXPERIENCE BASED EXAMPLES

### Real-time clinical experience sharing and adaptive learning

A system in which medical parameters entered to make a multi-parameter search are stored and become part in real-time of a shared anonym database. For example, if a case with a set of parameters (e.g. tumor type, histology, molecular profile and outcomes of previous treatments) is entered to make a search for similar cases, the data associated with case becomes instantly part of the shared clinical experience database. This means that the search results for a new patient by another user anywhere in the world are influenced in the next second. Further, the medical parameters of any patients can be dynamically updated. Medical parameters can be supplied for the system not only manual entry but via direct communications with electronic health records, laboratory equipment and wearable medical devices.

### Similarity based clinical experience ranking

A system in which clinical predictions (e.g, risk of disease, response to therapies, prognosis) are calculated based on clinical experiences with previous cases which are the most similar to the present case. The system automatically finds the group of cases with the highest number of matching parameters and aggregates experience of cases in this group or combines this experience with the experience of cases with less matching parameters with lower weight.
a) Similarity is defined by the number of shared medical (e.g. tumor type, molecular alterations, previous treatments) and environmental parameters (e.g. smoking).
b) The importance of a parameter is ranked based on biological relevance and frequency of that parameter, rare parameters having usually higher weight.
c) The unknown parameters are considered with a weight based on frequency of the unknown parameters in previous cases who share the same known both medical/genetic and environmental parameters.

### Automated treatment ranking based on collective clinical experience

As a specific use of the real-time clinical experience sharing and similarity based clinical experience ranking is the ranking of treatments based on their clinical efficacy in a group of cases, which are the most similar to the present case as described in the similarity based clinical experience ranking.

A specific use of the real-time clinical experience sharing, that the system automatically updates the frequency of molecular alterations, aggregates functional relevance (predictive or prognostic) to identify likely driver molecular genetic hotspot alterations, and links driver genes to target genes by learning from driver-target-drug clinical experiences. This enables the system to identify group of cases with the same driver genes and same activated drug-able pathways as in the present case.

### EXAMPLES 1 and 2

### Automated electronic real-time alerts of shared clinical experience

As a specific use of the real-time clinical experience sharing and treatment ranking, a registered user of the system can receive automatic electronic alerts (e.g. email, SMS) on a mobile device or computer when a similar case with a specific clinical experience is entered in the system. The user can set the threshold parameter to receive a personalized alert. The typical use of this function is when a patient and or his/her physician receive a notification that a patient with the same rare molecular alteration has been treated successfully with a specific treatment. The level of similarity (matching parameters) and level of treatment response (complete response, partial response, stable diseases, duration of progression free survival) can be set of the user.

### PUBLISHED EVIDENCE BASED EXAMPLES

### Automated treatment ranking with based on collective published evidence database

A system which ranks treatments based on aggregated published evidence which link the tumor type, histology, molecular alterations (biomarkers) to efficacy of therapies. The evidences are weighted based on the similarity between the parameters of the subjects or biological models in the published research and the present case. The importance of a parameter is ranked based on the biological relevance. For example, evidences related to the same molecular alteration have higher importance than evidences related to the same driver gene, same pathway (drug-able target), histology, tumor type or combination of these.

### EXAMPLE 3

### User developed, collective shared published evidence database

A system where evidences which link tumor types, histology, biomarkers, driver genes, targets and drugs can be added by a network of users to create published evidence based treatment ranking. The evidence database is automatically shared with other users using the same system.

### EXAMPLE 4

### Automated electronic real-time alerts of matching published evidence, drug registration or clinical trials

Users of the system can subscribe for automatic alerts:
a) when published evidence which are linked to the parameters of the case is entered in the system.
b) when conditions of the registration (and/or reimbursement conditions) of a novel drug match the parameters of the present case
c) when a new clinical trial with matching inclusion and exclusion criteria is entered is entered by any user of the system.

### EXAMPLE 1

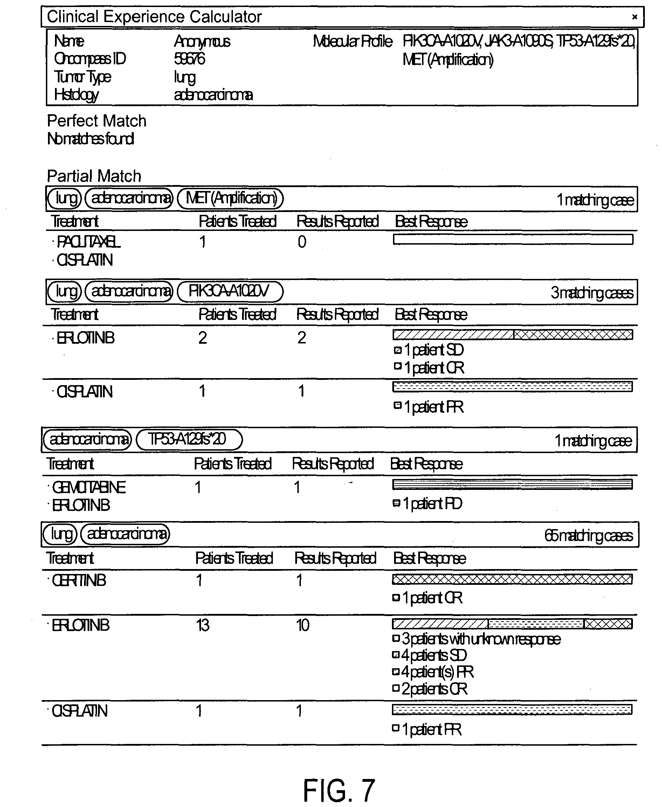

Referring to FIG. 7, a case of a 38 year old man with lung adenocarcinoma, PIK3CA-1020V, JAK3-A1090A, TP53-A129FS.20, MET amplification is entered in the system. The clinical experience calculator did not find patients, which perfectly match all known parameters. There is a group of 3 cases, which share the same tumor type, histology and one of the molecular alterations. Two cases with either of the other alterations and 65 cases with the same tumor type and histology independently from molecular alterations.

The first partial match was a patient with lung adenocarcinoma and MET amplification. The patient has received (or is receiving) Cisplatin + Paclitaxel chemotherapy, the best response is unknown. The clinical experience calculator shows the number of patients for whom a therapy was initiated, and lists in separate columns those, for whom a follow up information, that is, a therapeutic response has already been entered in the system. For this latter group, treatment outcomes are shown in color-coded bars. Therapies are ranked base on the best response they produce, with CR having the highest and PD having the lowest rank.

In the second partial match set, a stable disease (SD) and a complete response (CR) belongs to the Erlotinib therapy and a partial response (PR) to the Cisplatin therapy. Erlotinib comes first because there is at least one recorded CR for this therapy.

The clinical experience calculator, illustrated at the bottom portion of FIG. 7, shows also therapies belonging to the matching primary tumor site and histology type.

### EXAMPLE 2

### CLINICAL EXPERIENCE CALCULATOR

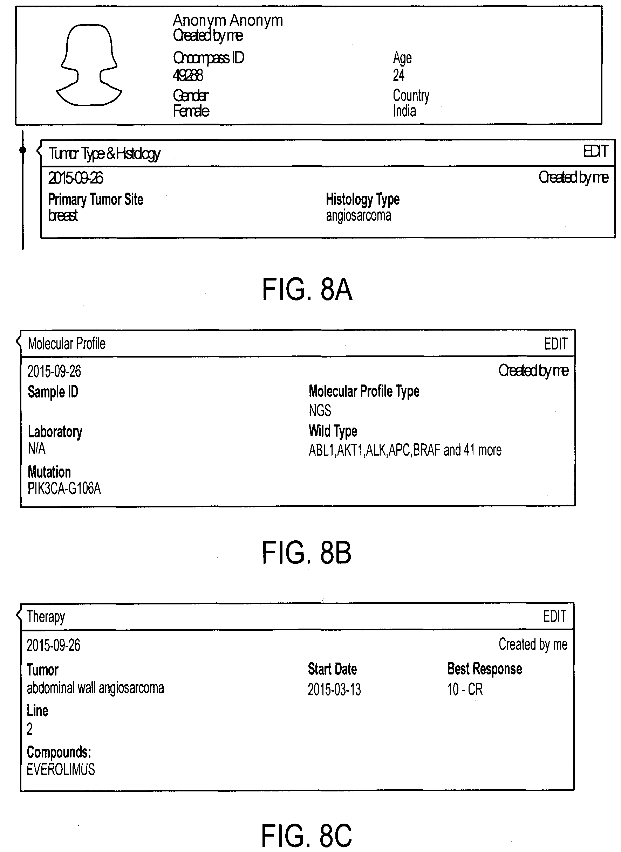

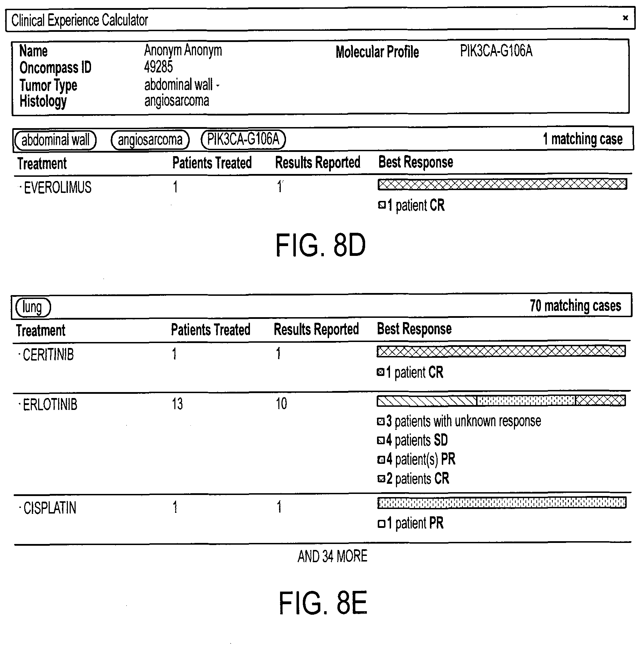

Referring to FIG. 8, the case of a 24 year old female patient with advanced angiosarcoma of the breast and the abdominal wall was diagnosed in India and her doctor entered her medical history, as illustrated in FIG. 8A.

As shown in FIG. 8B, the molecular profile analysis of the tumor found a rare mutation in the PIK3CA gene, the PIK3CA-G106A.

As shown in FIG. 8C, The patient received an mTOR inhibitor targeted therapy (e.g., Everolimus) which induced complete remission (CR) in the patient.

From this moment if another case is entered in the system the clinical experience calculator shows that that a previous case with the same tumor type, histology and molecular alteration has been successfully treated with a targeted therapy, as referenced in FIG. 8D.

This case has also contributed to our knowledge that this particular mutation is a driver mutation and in this tumor type mutations in PIK3CA predict sensitivity to mTOR inhibitors, as shown in FIG. 8E.

### EXAMPLE 3

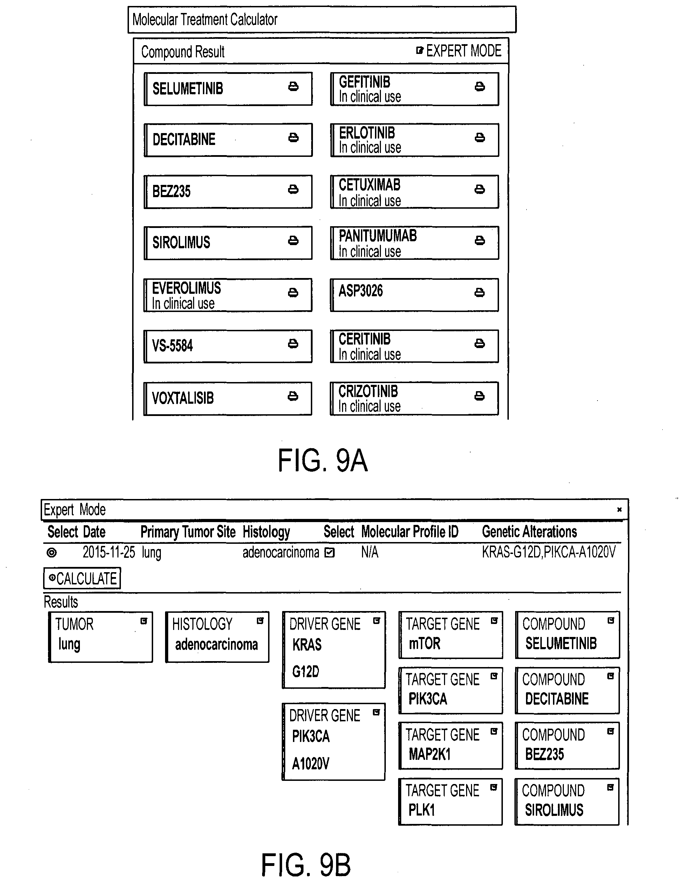

Referring to FIGS. 9A and 9B, the case of a 60 year old male with lung adenocarcinoma patients with KRAS-G12D and PIK3CA-A1020V is entered into the system. Treatment options are ranked based on aggregated evidences which link tumor type, histology and molecular alterations (positive/green, negative/red) to compounds.

The left column of FIG. 9A contains the positively associated compounds and the right column contains the negatively associated ones. The compound with the highest aggregated evidence level is the first in the list and the compound with the lowest aggregated evidence level is the last in the list.

Looking to FIG. 9B, the KRAS-G12D and the PIK3CA-A1020V mutations are both calculated to be driver mutations based on the evidence recorded in the system, thus their targets show up in the target column. In the last column, the compounds are listed which are associated with the drivers (directly or through their targets) or the tumor set (primary tumor site and histology type). For example, Selumetinib is a MEK inhibitor and the MEK is indirect target of the mutant KRAS.

### EXAMPLE 4

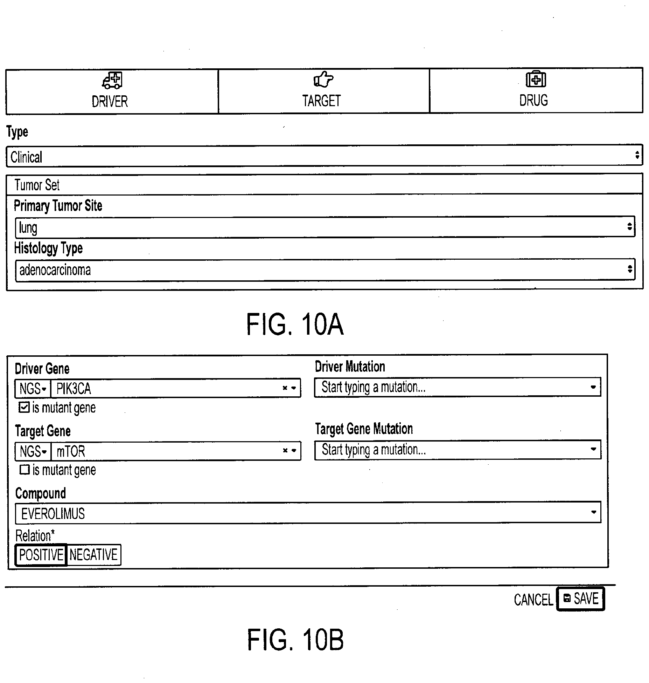

Referring to FIG. 10, evidence which links tumor type (lung), histology (adenocarcinoma), molecular alteration (PIK3CA mutation) , target (mTOR) and drug (e.g., Everolimus) is entered into the database.

The evidence database contains relations which can link the tumor set, the driver, the target and the compound with each other positively or negatively. These relations are mostly published in the scientific articles. In the Calculator, the types of associations mentioned above can be recorded in a user-friendly way, in a format that can be readily processed by the Calculator modules of the system.

### SIMILAR CASE CALCULATOR

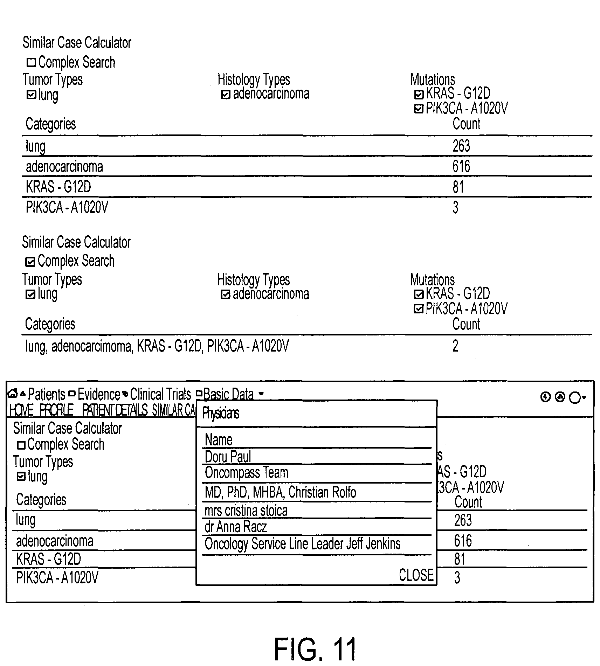

As illustrated in exemplary user interface of FIG. 11, in the system the user can search for patients with the same parameters separately or in complex searches for patients who match multiple parameters. If the user clicks on the number, the physician users who entered the case can be seen and contacted through a hyperlink, which leads to contact information or automatic email feature.

### TRIAL CALCULATOR

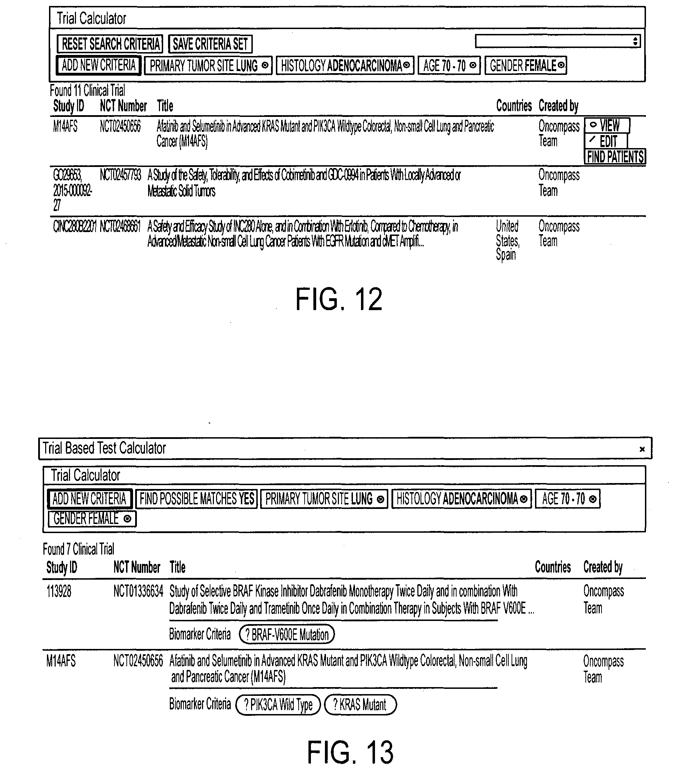

The trail calculator automatically import the inclusion and exclusion criteria from the medical history. The parameter sets can be saved, parameter can be turn off and then the original set off parameters can be restored. The Trial Calculator can be also used backward to find patients for a trial. An exemplary embodiment of the user interface of the Trial Calculator is illustrated in FIG. 12.

### TRIAL BASED TEST CALCULATOR

This calculator identifies clinical trials, which match the clinical exclusion and inclusion criteria and indicates which biomarkers have to be tested in addition as an exclusion or inclusion parameter. This tool can be used to support decision which diagnostic tests are important for the particular patient. An exemplary embodiment of the user interface of the Trial Calculator is illustrated in FIG. 13.

### TEST CALCULATOR

The Test Calculator ranks genetic alteration in order of frequency calculated based on the experience in patients in the database. The statistics are recalculated as more cases added. The calculator uses data generated in patients similar to the particular case (e.g. same tumor type, genetic alterations etc.)

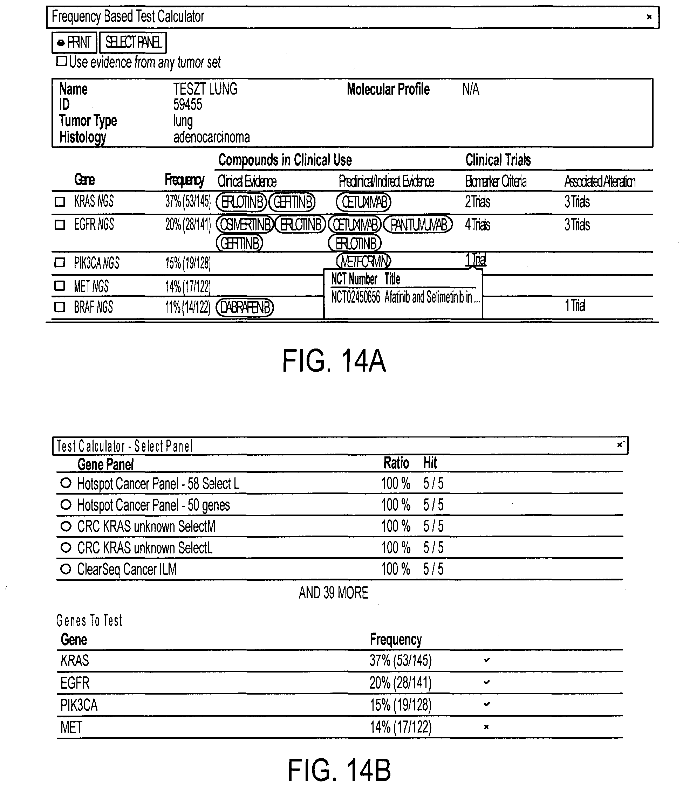

An exemplary user interface to the Test Calculator is illustrated in FIG. 14A. Next to the gene the potential clinical relevance is indicated. Compounds, which are linked based on scientific evidence to the gene (biomarker) are indicated (positive (green) or negative (red). In addition, clinical trials which match the parameters of the patient, which include the particular gene or biomarker to be tested as an inclusion or exclusion criteria, or testing a compound, which is associated with the gene is indicated.

Based on this information the genes, which have therapeutic relevance can be selected. The calculator list and ranks diagnostic panels in order of number of genes selected are in the panel. The selected genes are also listed again and indicated which one are covered by the selected panel. This tool can be used to select the panel which is sufficient, most cost effective for the patient. This tool can be used as a "webshop" to order diagnostic tests form different partner laboratories. An example of the user interface according to this aspect is illustrated in FIG. 14B.

### SUPERSEARCH

In this tool multiple parameters can be freely entered in the search box without registering a case. This tool enables quick searches (calculations) and research in the database.

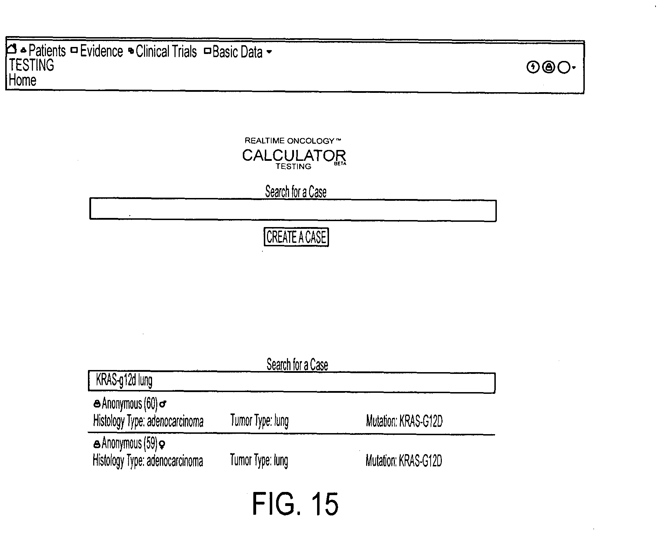

All calculators can have a SUPERSEARCH version. For example, entering the gene mutation and the tumor type can immediately show the best effective therapies in patients with this gene mutation and tumor type. The same way patients, cases can be quickly found in the SUPERSEARCH. An illustration of a SUPERSEARCH feature is provided in FIG. 15.

### SOLUTIONS

In the next phase of the history of oncology, the clinical experience of oncologists and all clinicians treating cancer patients will have a greater significance than it was during the age of large phase III clinical trials. The results of off label treatments are often reported in case studies. These reports are getting more and more important in the molecular profile based personalized medicine to gather clinical evidence. The back-draw is the publication bias to publish only positive cases. The negative results should be published as well to avoid future unsuccessful treatment in seemingly "obvious" indications. In both positive and negative case reports, we have to remember that the association between molecular profile and response is only "proven" for tumors with an exactly same profile. Therefore, as much as possible detailed molecular profiling of tumors should be performed and reported together with the clinical outcome.

Referring the patient a molecular matching clinical trial, if available has a lot of obvious advantages over individual off label treatments, and can provide access to treatments not in clinical use yet.

Fortunately, the detailed molecular pharmacological studies and the integrated companion diagnostics development is a standard procedure of the process of preclinical anticancer discovery today.

Many patients today have detailed molecular profile, which can be used to decide if there is a matching trial or not. Phase I trials are rarely recommended due to lack of previous clinical evidence and lack of established dose. However, in case of molecular targeted trials and matching profile even phase I trials can provide a significant benefit for the patients. These trials are also called phase "0" trials to distinguish them from phase I-s, which only aim to find the maximal tolerated dose. Phase "0" tests the safety (Ia) and efficacy (Ib) in a subgroup of patients with a specific molecular profile, which is most likely associated with the efficacy of the drug candidate based on the results of the preclinical studies and our understanding molecular cancer biology. These trials sometimes but not always are also restricted to the histology type or localization of the tumor.

It is often debated whether an unselected patient population should be treated first and then to conduct a retrospective biomarker analysis to find the best responders. The biomarker than can be further validated in prospective trials. The advantage is that we do not run the risk of wrong preclinical hypothesis. On the other hand, the competition for patients available for clinical trials is so great that this approach would slow down clinical developments. Further, drug developers should find the niche of their drug as fast as possible for the lowest cost to ensure the longest competition free lifespan of the drug on the market. Today, the goal is not to find the largest group where the drug has some benefit "to find the patients for the drug", but to find patients who like benefit from the drug more than any other possible treatment.

In case of breakthrough results the FDA is very open to give fast track marketing approval. The new element would be to give approval for all tumor types with the same molecular profile. The efficacy of the drugs could be monitored in post marketing phase IV clinical trials and reported to the FDA. Based on experience, the FDA may restrict the use of the drug in tumor types where results were not satisfactory. This approach will help all patients with a particular molecular to access the matching targeted drugs right away. If the drug is only in clinical trial (phase 0) the tumor type does not restrict the access to the trial, therefore all patients who progressed all standard therapy can participate. After this short clinical phase all patients with the matching can access the drug. This way physicians can rely also molecular evidence when they wish to find the best therapy for their patients. In this better future, we do not have to change the "gas pump" in patients if we know that the "sparking plug" is broken.

The invention can be used any medical field where: a) the disease or diseases show large inter-patient heterogeneity in prognosis, drug sensitivity and side effects; b) there are already available diagnostic methods, which generate standardized data to detect these differences; c) there are more and more treatments options. In this case, the invention can help to associate the multiple sub-group of patients identified with diagnostic methods with the right treatments.

The most obvious such medical field is cancer, which show great inter-patient heterogeneity, which can be explained by the underlying molecular heterogeneity, which can be detected with molecular diagnostics methods which generate standardized data, and where hundreds of novel therapies are being developed.

But many other human (and animal) disease show clinical heterogeneity, which have molecular genetic (and epigenetic) background, which can be detected today. In addition, there are novel methods of high throughput diagnostics, for example metabolomics, which will revolutionize medicine. As these methods become common, there will be a large un-met medical need to link the newly discovered disease subtypes to treatments where this invention will be useful.

The whole approach to use multiple diagnostics "omics" data for personalized treatment is called precision medicine. The present invention is a system to advance precision medicine. And as such it can be called a personal "precision medicine calculator".

## Claims

1. An adaptive medical treatment decision system for targeted cancer therapy, comprising:
a medical experience register, the medical experience register storing medical experience data from a plurality of users, wherein the medical experience data from each user is encoded and includes anonymous, patient-specific physical, biological and clinical data and;
a clinical evidence register, the clinical evidence register storing result data from at least a subset of the plurality of users, the result data including anonymous molecular and clinical profiles of respective prior patients, diagnoses of the respective prior patients, treatments administered to the respective prior patients, the molecular variations found in the patient's cancer, frequency of genetic variations, drug targets, and outcomes of the treatments administered to the respective prior patients in association with specific genetic alterations;
a processor connected to the medical experience register and the clinical evidence register for requesting and receiving signals from the medical experience register and the clinical evidence register, the processor enabled to process said received signals to rank treatment options based on a specific patient physical, biological or clinical profile and diagnosis and frequency of genetic variations stored in the clinical evidence register,
wherein, in the therapy of cancer, targeted therapies are ranked based on their response in previously treated patients similar to the present patient,
wherein the ranking prefers the experience associated with a targeted therapy with patients with the same driver genetic alterations selected from the group comprising point mutation, copy number variations, translocations;
and if there is no experience yet with the exactly same driver genetic alterations, the targeted therapies are ranked based on the clinical results in patients with alterations in the same driver genes,
and if there is no experience yet with the exactly same driver genes, experience with driver genes in the same signal transduction pathway, therefore the same molecular targets, is considered,
and if there is no experience yet with the same targets, the ranking is based on the tumor type;
wherein said patient is to be treated based on this ranking and the clinical response is registered in the system to add to the experience database.

2. A method of assigning preference rank to treatment options for a specific patient, comprising:
continually ranking a plurality of treatments for a plurality of corresponding diseases, said ranking based on a plurality of information specific to each of a plurality of patients, wherein said information includes diagnosis, genetic profile, genetic variations, treatment and outcome for each patient of the plurality of patients and frequency of genetic variations, said continual ranking performed by a special processor for receiving the plurality of information and corresponding metadata;
receiving at the special processor specific descriptors of the specific patient's condition, the specific descriptors including a diagnosis and a specific genetic profile of the specific patient;
the special processor generating at least one treatment profile based on the continual ranking of the plurality of treatments, the at least one treatment profile having a high ranking for the specific patient based on the specific descriptors at the time the at least one treatment profile is generated;
wherein, in the therapy of cancer, targeted therapies are ranked based on their response rate in previously treated patients similar to the present patient,
wherein the ranking prefers the experience with patients with the same driver genetic alterations selected from the group comprising point mutation, copy number variations, translocations;
and if there is no experience yet with the exactly same driver genetic alterations, the targeted therapies are ranked based on the clinical results in patients with alterations in the same driver genes,
and if there is no experience yet with the exactly same driver genes, experience with driver genes in the same signal transduction pathway, therefore the same molecular targets, is considered,
and if there is no experience yet with the same targets, the ranking is based on the tumor type;
after treating said patient according to the at least one treatment profile based on the ranking registering the clinical response in the system to add to the experience database.

3. The method of claim 2, wherein if there is/are parameters that are a genetic variation which can alter the drug sensitivity, but is/are unknown in the particular patient, the treatment rank is based on the frequency of the unknown parameter based on experience with patients with similar characteristics in the shared database or register.

4. The method of claim 2, further comprising receiving at the special processor a treatment selected for the specific patient and outcome of the treatment of the specific patient, wherein the treatment selected for the specific patient and the outcome of the treatment for the specific patient influence the continual ranking of treatment for a corresponding disease.

5. The method of claim 2, wherein said information includes laboratory test results for at least some of the plurality of patients.

6. The method of claim 5, wherein said specific descriptors include patient-specific laboratory test results.

7. The method of claim 2, wherein said information includes data generated by diagnostic test equipment received directly from the diagnostic test equipment.

8. The method of claim 7, wherein said specific descriptors include patient-specific diagnostic test results.

9. The method of claim 2, wherein the ranking of the therapies is both based on the clinical experience of the same group of patients having the same molecular profile, same driver genes and/or same tumor type, and evidence, including published and system generated evidence supporting the most likely driver, target and drug.

10. The method of claim 2, wherein the clinical predictions for response to therapies or prognosis are calculated based on clinical experiences with previous cases which are the most similar to the present case, wherein the system automatically finds the group of cases with the highest number of matching parameters and aggregates experience of cases in this group or combines this experience with the experience of cases with less matching parameters with lower weight.

11. The method of claim 10, wherein
a) similarity is defined by the number of shared medical parameters selected from the group comprising tumor type, molecular alterations, and previous treatments, and environmental parameters, and
b) importance of a parameter is ranked based on biological relevance and frequency of that parameter, and
c) the unknown parameters are considered with a weight based on frequency of the unknown parameters in previous cases who share the same known both medical/genetic and environmental parameters.

12. The system of claim 1, wherein if there is/are parameters that are a genetic variation which can alter the drug sensitivity, but is/are unknown in the particular patient, the treatment rank is based on the frequency of the unknown parameter based on experience with patients with similar characteristics in the shared database or register.

13. The system of claim 1, wherein the ranking of the therapies is both based on the clinical experience of the same group of patients having the same molecular profile, same driver genes and/or same tumor type, and evidence, including published and system generated evidence supporting the most likely driver, target and drug.

14. The system of claim 1, wherein the clinical predictions for response to therapies or prognosis are calculated based on clinical experiences with previous cases which are the most similar to the present case, wherein the system automatically finds the group of cases with the highest number of matching parameters and aggregates experience of cases in this group or combines this experience with the experience of cases with less matching parameters with lower weight.

15. The system of claim 1, wherein
a) similarity is defined by the number of shared medical parameters selected from the group comprising tumor type, molecular alterations, and previous treatments, and environmental parameters, and
b) importance of a parameter is ranked based on biological relevance and frequency of that parameter, and
c) the unknown parameters are considered with a weight based on frequency of the unknown parameters in previous cases who share the same known both medical/genetic and environmental parameters.

## Patentansprüche

1. Adaptives medizinisches Behandlungsentscheidungssystem für eine gezielte Krebstherapie, umfassend:
ein Register medizinischer Erfahrung, wobei das Register medizinischer Erfahrung Daten medizinischer Erfahrung von einer Vielzahl von Benutzern speichert, wobei die Daten medizinischer Erfahrung von jedem Benutzer codiert sind und anonyme, patientenspezifische physikalische, biologische und klinische Daten einschließen, und;
ein Register klinischer Nachweise, wobei das Register klinischer Nachweise Ergebnisdaten von mindestens einer Teilmenge der Vielzahl von Benutzern speichert, die Ergebnisdaten anonyme molekulare und klinische Profile jeweiliger früherer Patienten, Diagnosen der jeweiligen früheren Patienten, an die jeweiligen früheren Patienten verabreichten Behandlungen, die bei der Krebserkrankung des Patienten festgestellten molekularen Variationen, eine Häufigkeit genetischer Variationen, Arzneimittelziele und Wirkungen der an die jeweiligen früheren Patienten verabreichten Behandlungen im Zusammenhang mit spezifischen genetischen Veränderungen einschließen;
einen Prozessor, der mit dem Register medizinischer Erfahrung und dem Register klinischer Nachweise zum Anfordern und Empfangen von Signalen von dem Register medizinischer Erfahrung und dem Register klinischer Nachweise verbunden ist, wobei der Prozessor fähig ist, die empfangenen Signale zu verarbeiten, um Behandlungsoptionen basierend auf einem spezifischen physikalischen, biologischen oder klinischen Patientenprofil und einer Diagnose und der Häufigkeit genetischer Variationen, die in dem Register klinischer Nachweise gespeichert sind, einzustufen,
wobei bei der Therapie von Krebs gezielte Therapien basierend auf ihrer Reaktion bei zuvor behandelten Patienten, die dem vorliegenden Patienten ähnlich sind, eingestuft werden,
wobei die Einstufung die Erfahrung bevorzugt, die mit einer gezielten Therapie bei Patienten mit den gleichen genetischen Treiberveränderungen zusammenhängt, die aus der Gruppe ausgewählt sind, umfassend Punktmutation, Kopienzahlvariationen, Translokationen;
und falls es noch keine Erfahrung mit den genau gleichen genetischen Treiberveränderungen gibt, die gezielten Therapien basierend auf den klinischen Ergebnissen bei Patienten mit Veränderungen in den gleichen Treibergenen eingestuft werden,
und falls es noch keine Erfahrung mit den genau gleichen Treibergenen gibt, die Erfahrung mit Treibergenen in dem gleichen Signalübertragungsweg, also den gleichen molekularen Zielen, berücksichtigt wird,
und falls es noch keine Erfahrung mit den gleichen Zielen gibt, die Einstufung basierend auf der Tumorart erfolgt;
wobei der Patient basierend auf dieser Einstufung zu behandeln ist und die klinische Reaktion in dem System registriert wird, um diese der Erfahrungsdatenbank hinzuzufügen.

2. Verfahren zum Zuweisen einer Präferenzeinstufung zu Behandlungsoptionen für einen spezifischen Patienten, umfassend:
kontinuierliches Einstufen einer Vielzahl von Behandlungen für eine Vielzahl von entsprechenden Krankheiten, wobei die Einstufung auf einer Vielzahl von Informationen basiert, die für jeden einer Vielzahl von Patienten spezifisch sind, wobei die Informationen die Diagnose, das genetische Profil, genetische Variationen, die Behandlung und die Wirkung für jeden Patienten der Vielzahl von Patienten und die Häufigkeit genetischer Variationen einschließen, wobei die kontinuierliche Einstufung durch einen speziellen Prozessor zum Empfangen der Vielzahl von Informationen und der entsprechenden Metadaten durchgeführt wird;
Empfangen spezifischer Deskriptoren des Zustands des spezifischen Patienten an dem besonderen Prozessor, wobei die spezifischen Deskriptoren eine Diagnose und ein spezifisches genetisches Profil des spezifischen Patienten einschließen;
wobei der besondere Prozessor mindestens ein Behandlungsprofil basierend auf der fortlaufenden Einstufung der Vielzahl von Behandlungen erzeugt, wobei das mindestens eine Behandlungsprofil eine hohe Einstufung für den spezifischen Patienten basierend auf den spezifischen Deskriptoren zu dem Zeitpunkt, zu dem das mindestens eine Behandlungsprofil erzeugt wird, aufweist;
wobei bei der Therapie von Krebs gezielte Therapien basierend auf ihrer Reaktionsrate bei zuvor behandelten Patienten, die dem vorliegenden Patienten ähnlich sind, eingestuft werden,
wobei die Einstufung die Erfahrung bei Patienten mit den gleichen genetischen Treiberveränderungen bevorzugt, die aus der Gruppe ausgewählt sind, umfassend Punktmutation, Kopienzahlvariationen, Translokationen;
und falls es noch keine Erfahrung mit den genau gleichen genetischen Treiberveränderungen gibt, die gezielten Therapien basierend auf den klinischen Ergebnissen bei Patienten mit Veränderungen in den gleichen Treibergenen eingestuft werden,
und falls es noch keine Erfahrung mit den genau gleichen Treibergenen gibt, die Erfahrung mit Treibergenen in dem gleichen Signalübertragungsweg, also den gleichen molekularen Zielen, berücksichtigt wird,
und falls es noch keine Erfahrung mit den gleichen Zielen gibt, die Einstufung basierend auf der Tumorart erfolgt;
nach dem Behandeln des Patienten gemäß mindestens einem Behandlungsprofil basierend darauf, dass die Einstufung die klinische Reaktion in dem System registriert, um diese der Erfahrungsdatenbank hinzuzufügen.

3. Verfahren nach Anspruch 2, wobei, falls es (einen) Parameter gibt, bei denen es sich um eine genetische Variation handelt, die die Arzneimittelempfindlichkeit verändern kann, der/die jedoch bei dem speziellen Patienten unbekannt ist/sind, die Behandlungseinstufung auf der Häufigkeit des unbekannten Parameters basiert, basierend auf der Erfahrung mit Patienten mit ähnlichen Merkmalen in der gemeinsam genutzten Datenbank oder dem gemeinsam genutzten Register.

4. Verfahren nach Anspruch 2, ferner umfassend das Empfangen einer Behandlung, die für den spezifischen Patienten ausgewählt ist, und der Wirkung der Behandlung des spezifischen Patienten an dem besonderen Prozessor, wobei die Behandlung, die für den spezifischen Patienten ausgewählt ist, und die Wirkung der Behandlung für den spezifischen Patienten die kontinuierliche Einstufung der Behandlung für eine entsprechende Krankheit beeinflussen.

5. Verfahren nach Anspruch 2, wobei die Informationen Labortestergebnisse für mindestens einige der Vielzahl von Patienten einschließen.

6. Verfahren nach Anspruch 5, wobei die spezifischen Deskriptoren patientenspezifische Labortestergebnisse einschließen.

7. Verfahren nach Anspruch 2, wobei die Informationen Daten einschließen, die durch Diagnosetestausrüstung erzeugt werden, die von der Diagnosetestausrüstung direkt empfangen werden.

8. Verfahren nach Anspruch 7, wobei die spezifischen Deskriptoren patientenspezifische diagnostische Testergebnisse einschließen.

9. Verfahren nach Anspruch 2, wobei die Einstufung der Therapien sowohl auf der klinischen Erfahrung der gleichen Gruppe von Patienten, die das gleiche molekulare Profil, die gleichen Treibergene und/oder die gleiche Tumorart aufweisen, als auch auf Nachweisen, einschließlich veröffentlichten und systemerzeugten Nachweisen, die den wahrscheinlichsten Treiber, das Ziel und das Arzneimittel unterstützen, basiert.

10. Verfahren nach Anspruch 2, wobei die klinischen Vorhersagen für die Reaktion auf Therapien oder die Prognose basierend auf klinischen Erfahrungen mit früheren Fällen berechnet werden, die dem vorliegenden Fall am ähnlichsten sind, wobei das System die Gruppe von Fällen mit der höchsten Anzahl übereinstimmender Parameter automatisch findet und die Erfahrung der Fälle in dieser Gruppe aggregiert oder diese Erfahrung mit der Erfahrung von Fällen mit weniger übereinstimmenden Parametern mit geringerer Gewichtung kombiniert.

11. Verfahren nach Anspruch 10, wobei
a) die Ähnlichkeit durch die Anzahl gemeinsamer medizinischer Parameter definiert wird, die aus der Gruppe ausgewählt werden, umfassend die Tumorart, molekulare Veränderungen und vorherige Behandlungen und umweltbedingte Parameter, und
b) eine Wichtigkeit eines Parameters basierend auf biologischer Relevanz und Häufigkeit dieses Parameters eingestuft wird, und
c) die unbekannten Parameter mit einer Gewichtung berücksichtigt werden, die auf der Häufigkeit der unbekannten Parameter in früheren Fällen basiert, die die gleichen bekannten sowohl medizinischen/genetischen als auch umweltbedingten Parameter teilen.

12. System nach Anspruch 1, wobei, falls es (einen) Parameter gibt, bei denen es sich um eine genetische Variation handelt, die die Arzneimittelempfindlichkeit verändern kann, der/die jedoch bei dem speziellen Patienten unbekannt ist/sind, die Behandlungseinstufung auf der Häufigkeit des unbekannten Parameters basiert, basierend auf der Erfahrung mit Patienten mit ähnlichen Merkmalen in der gemeinsam genutzten Datenbank oder dem gemeinsam genutzten Register.

13. System nach Anspruch 1, wobei die Einstufung der Therapien sowohl auf der klinischen Erfahrung der gleichen Gruppe von Patienten, die das gleiche molekulare Profil, die gleichen Treibergene und/oder die gleiche Tumorart aufweisen, als auch auf Nachweisen, einschließlich veröffentlichten und systemerzeugten Nachweisen, die den wahrscheinlichsten Treiber, das Ziel und das Arzneimittel unterstützen, basiert.

14. System nach Anspruch 1, wobei die klinischen Vorhersagen für die Reaktion auf Therapien oder die Prognose basierend auf klinischen Erfahrungen mit früheren Fällen berechnet werden, die dem vorliegenden Fall am ähnlichsten sind, wobei das System die Gruppe von Fällen mit der höchsten Anzahl übereinstimmender Parameter automatisch findet und die Erfahrung der Fälle in dieser Gruppe aggregiert oder diese Erfahrung mit der Erfahrung von Fällen mit weniger übereinstimmenden Parametern mit geringerer Gewichtung kombiniert.

15. System nach Anspruch 1, wobei
a) die Ähnlichkeit durch die Anzahl gemeinsamer medizinischer Parameter definiert wird, die aus der Gruppe ausgewählt werden, umfassend die Tumorart, molekulare Veränderungen und vorherige Behandlungen und umweltbedingte Parameter, und
b) eine Wichtigkeit eines Parameters basierend auf biologischer Relevanz und Häufigkeit dieses Parameters eingestuft wird, und
c) die unbekannten Parameter mit einer Gewichtung berücksichtigt werden, die auf der Häufigkeit der unbekannten Parameter in früheren Fällen basiert, die die gleichen bekannten sowohl medizinischen/genetischen als auch umweltbedingten Parameter teilen.

## Revendications

1. Système adaptatif de décision de traitement médical pour une thérapie ciblée du cancer, comprenant :
un registre d'expériences médicales, le registre d'expériences médicales stockant des données d'expériences médicales provenant d'une pluralité d'utilisateurs, dans lequel les données d'expériences médicales de chaque utilisateur sont codées et comportent des données physiques, biologiques et cliniques anonymes et spécifiques à un patient et ;
un registre de preuves cliniques, le registre de preuves cliniques stockant des données de résultats provenant d'au moins un sous-ensemble de la pluralité d'utilisateurs, les données de résultats comportant les profils moléculaires et cliniques anonymes de patients antérieurs respectifs, les diagnostics des patients antérieurs respectifs, les traitements administrés aux patients antérieurs respectifs, les variations moléculaires trouvées dans le cancer des patients, la fréquence des variations génétiques, les cibles de médicaments, et les résultats des traitements administrés aux patients antérieurs respectifs en association avec des altérations génétiques spécifiques ;
un processeur connecté au registre d'expériences médicales et au registre de preuves cliniques permettant de demander et recevoir des signaux du registre d'expériences médicales et du registre de preuves cliniques, le processeur étant capable de traiter lesdits signaux reçus pour classer des options de traitement sur la base d'un profil physique, biologique ou clinique, du diagnostic et de la fréquence des variations génétiques d'un patient spécifique stockés dans le registre de preuves cliniques,
dans lequel, dans le traitement du cancer, les thérapies ciblées sont classées sur la base de leur réponse chez des patients traités antérieurement et similaires au patient actuel,
dans lequel le classement préfère l'expérience associée à une thérapie ciblée avec des patients ayant les mêmes altérations génétiques motrices choisies dans le groupe comprenant des mutations ponctuelles, des variations du nombre de copies, des translocations ;
et s'il n'y a pas encore d'expérience avec exactement les mêmes altérations génétiques motrices, les thérapies ciblées sont classées sur la base des résultats cliniques chez des patients ayant des altérations dans les mêmes gènes moteurs,
et s'il n'y a pas encore d'expérience avec exactement les mêmes gènes moteurs, l'expérience avec des gènes moteurs dans la même voie de transduction de signal, donc les mêmes cibles moléculaires, est prise en compte,
et s'il n'y a pas encore d'expérience avec les mêmes cibles, le classement est basé sur le type de tumeur ; dans lequel le patient doit être traité sur la base de ce classement et la réponse clinique est enregistrée dans le système pour l'ajouter à la base de données d'expériences.

2. Procédé d'attribution d'un classement de préférence à des options de traitement pour un patient spécifique, comprenant :
le classement continu d'une pluralité de traitements pour une pluralité de maladies correspondantes, ledit classement étant basé sur une pluralité d'informations spécifiques à chacun d'une pluralité de patients, dans lequel lesdites informations comportent le diagnostic, le profil génétique, les variations génétiques, le traitement et le résultat pour chaque patient de la pluralité de patients et la fréquence des variations génétiques, ledit classement continu étant effectué par un processeur spécial permettant de recevoir la pluralité d'informations et les métadonnées correspondantes ;
la réception, au niveau du processeur spécial, de descripteurs spécifiques de l'état du patient spécifique, les descripteurs spécifiques comportant un diagnostic et un profil génétique spécifique du patient spécifique ;
le processeur spécial générant au moins un profil de traitement sur la base du classement continu de la pluralité de traitements, l'au moins un profil de traitement ayant un classement élevé pour le patient spécifique sur la base des descripteurs spécifiques au moment où l'au moins un profil de traitement est généré ;
dans lequel, dans le traitement du cancer, les thérapies ciblées sont classées sur la base de leur taux de réponse chez des patients traités antérieurement et similaires au patient actuel,
dans lequel le classement préfère l'expérience avec des patients ayant les mêmes altérations génétiques motrices choisies dans le groupe comprenant des mutations ponctuelles, des variations du nombre de copies, des translocations ;
et s'il n'y a pas encore d'expérience avec exactement les mêmes altérations génétiques motrices, les thérapies ciblées sont classées sur la base des résultats cliniques chez des patients ayant des altérations dans les mêmes gènes moteurs,
et s'il n'y a pas encore d'expérience avec exactement les mêmes gènes moteurs, l'expérience avec des gènes moteurs dans la même voie de transduction de signal, donc les mêmes cibles moléculaires, est prise en compte,
et s'il n'y a pas encore d'expérience avec les mêmes cibles, le classement est basé sur le type de tumeur ;
après avoir traité ledit patient conformément à l'au moins un profil de traitement sur la base du classement, l'enregistrement de la réponse clinique dans le système pour l'ajouter à la base de données d'expériences.

3. Procédé selon la revendication 2, dans lequel, s'il existe un ou des paramètres qui sont une variation génétique qui peut modifier la sensibilité aux médicaments, mais qui est ou sont inconnus chez le patient en question, le classement du traitement est basé sur la fréquence du paramètre inconnu, sur la base de l'expérience acquise avec des patients ayant des caractéristiques similaires dans la base de données ou le registre partagé.

4. Procédé selon la revendication 2, comprenant en outre la réception, au niveau du processeur spécial, d'un traitement choisi pour le patient spécifique et du résultat du traitement du patient spécifique, dans lequel le traitement choisi pour le patient spécifique et le résultat du traitement pour le patient spécifique influencent le classement continu du traitement pour une maladie correspondante.

5. Procédé selon la revendication 2, dans lequel lesdites informations comportent des résultats de tests de laboratoire pour au moins une partie de la pluralité de patients.

6. Procédé selon la revendication 5, dans lequel lesdits descripteurs spécifiques comportent des résultats de tests de laboratoire spécifiques au patient.

7. Procédé selon la revendication 2, dans lequel lesdites informations comportent des données générées par des équipements de test diagnostique reçues directement des équipements de test diagnostique.

8. Procédé selon la revendication 7, dans lequel lesdits descripteurs spécifiques comportent des résultats de tests diagnostiques spécifiques au patient.

9. Procédé selon la revendication 2, dans lequel le classement des thérapies est basé à la fois sur l'expérience clinique du même groupe de patients ayant le même profil moléculaire, les mêmes gènes moteurs et/ou le même type de tumeur, et sur des preuves, y compris des preuves publiées et générées par le système, soutenant le moteur, la cible et le médicament les plus probables.

10. Procédé selon la revendication 2, dans lequel les prédictions cliniques pour la réponse aux thérapies ou le pronostic sont calculées sur la base d'expériences cliniques de cas précédents qui sont les plus similaires au cas actuel, dans lequel le système, de manière automatique, trouve le groupe de cas ayant le plus grand nombre de paramètres de correspondance et agrège l'expérience des cas dans ce groupe ou combine cette expérience avec l'expérience des cas ayant moins de paramètres de correspondance avec une pondération plus faible.

11. Procédé selon la revendication 10, dans lequel
a) la similarité est définie par le nombre de paramètres médicaux communs choisis dans le groupe comprenant le type de tumeur, les altérations moléculaires, et les traitements antérieurs, et les paramètres environnementaux, et
b) l'importance d'un paramètre est classée sur la base de sa pertinence biologique et de la fréquence de ce paramètre, et
c) les paramètres inconnus sont pris en compte avec une pondération basée sur la fréquence des paramètres inconnus dans les cas précédents qui partagent les mêmes paramètres médicaux/génétiques et environnementaux connus.

12. Système selon la revendication 1, dans lequel, s'il existe un ou des paramètres qui sont une variation génétique qui peut modifier la sensibilité aux médicaments, mais qui est ou sont inconnus chez le patient en question, le classement du traitement est basé sur la fréquence du paramètre inconnu, sur la base de l'expérience acquise avec des patients ayant des caractéristiques similaires dans la base de données ou le registre partagé.

13. Système selon la revendication 1, dans lequel le classement des thérapies est basé à la fois sur l'expérience clinique du même groupe de patients ayant le même profil moléculaire, les mêmes gènes moteurs et/ou le même type de tumeur, et sur des preuves, y compris des preuves publiées et générées par le système, soutenant le moteur, la cible et le médicament les plus probables.

14. Système selon la revendication 1, dans lequel les prédictions cliniques pour la réponse aux thérapies ou le pronostic sont calculées sur la base d'expériences cliniques de cas précédents qui sont les plus similaires au cas actuel, dans lequel le système, de manière automatique, trouve le groupe de cas ayant le plus grand nombre de paramètres de correspondance et agrège l'expérience des cas dans ce groupe ou combine cette expérience avec l'expérience des cas ayant moins de paramètres de correspondance avec une pondération plus faible.

15. Système selon la revendication 1, dans lequel
a) la similarité est définie par le nombre de paramètres médicaux communs choisis dans le groupe comprenant le type de tumeur, les altérations moléculaires, et les traitements antérieurs, et les paramètres environnementaux, et
b) l'importance d'un paramètre est classée sur la base de sa pertinence biologique et de la fréquence de ce paramètre, et
c) les paramètres inconnus sont pris en compte avec une pondération basée sur la fréquence des paramètres inconnus dans les cas précédents qui partagent les mêmes paramètres médicaux/génétiques et environnementaux connus.
